# EUROPEAN PATENT APPLICATION

(11) **EP 4 588 345 A1**
(43) Date of publication of application: **23.07.2025**
(21) Application number: 25152445.0
(22) Date of filing: 17.01.2025
(51) Int. Cl.: A01K 67/0276, A01K 67/0278, C07K 16/00, C12N 15/113, C12N 15/85

(54) **GENETICALLY MODIFIED NON-HUMAN MAMMALS, PREPARATION METHODS THEREFOR, AND APPLICATIONS THEREOF**

(30) Priority: 19.01.2024 CN 202410082783
(71) Applicant: Rengene Biotechnology Co., Ltd., Beijing (CN); Beijing Life Biosciences Co.,LTD, Beijing (CN)
(72) Inventor: WANG, Haoyi, Beijing (CN); DU, Xiaoming, Beijing (CN); GAO, Hui, Beijing (CN); ZHU, Pingxia, Beijing (CN); WU, Yi, Beijing (CN)
(74) Representative: LLR

(57) **Abstract**

The present invention provides a method for preparing a genetically modified non-human mammal for producing a humanized antibody as well as use thereof, which method comprises: (1) bringing about a disruption of an endogenous heavy chain immunoglobulin gene loci in a non-human mammal; and (2) introducing a human IGHV gene, a human IGHD gene, a human IGHJ gene as well as an endogenous IgHG2c gene, IgHE gene, IgHA gene, and LCR region of the non-human mammal into the non-human mammal obtained in step (1). The non-human mammal prepared according to the method of the present invention, after antigen immunization, enables efficiently producing a humanized whole antibody or single heavy chain antibody or nanobody with high immune titer. Furthermore, the method of the present invention enables obtaining a positive animal with high efficiency, such that humanized mice with different antibody diversities can be readily prepared, thereby realizing the nanobody sequence diversity using a variety of strains of mice.

## Description

### Cross-reference to Related Application

The present application claims the priority of Chinese Patent Application No. 202410082783.6, filed on January 19, 2024, and entitled "Method for Preparing Genetically Modified Non-human Mammal and Application Thereof', the entire contents of which are incorporated herein by reference.

### Technical Field

The present invention belongs to the field of biotechnology, and in particular relates to a method for preparing a genetically modified non-human mammal and use thereof.

### Background Art

In camelids and cartilaginous fish, a uniquely structured antibody, called heavy-chain-only antibody, occurs naturally. This antibody consists of only heavy chains and its variable region fragment has a molecular weight of 15 kDa, about one-tenth that of a conventional antibody which is 150-160 kDa. The variable region fragment of a heavy-chain-only antibody is also known as a nanobody. Nanobodies have been widely used in the development of bispecific/multispecific antibodies, in the CAR-T cell therapy, etc. As of January 2023, four nanobody-based therapeutics have been marketed, among which the nanobody-based BCMA CAR-T therapy developed by Legend Bio has achieved excellent clinical results. In addition, more than 10 nanobody molecules developed as neutralizing antibodies have entered clinical phase II/III. The drug development and application of nanobodies are still in a relatively early stage, which shows great application potential and development prospects.

Fully human nanobody-producing mice are useful for the development of nanobody drugs. Based on the independently developed humanization technology for genome fragments at a level ranging from hundreds of kilobase pairs to mega base pairs (abbreviated as MB), humanization of heavy chain genes of antibodies, encompassing the major human heavy chain variable region genes, has been achieved. HuNano Mouse enables direct screening of therapeutic antibody gene sequences for serious diseases, such as fully human nanobodies, bifunctional antibodies, and those for CAR-T therapy. The produced fully human nanobody sequences can be used for drug development without further in vitro humanization, thereby saving a lot of time and cost and reducing the risk associated with subsequent drug development. The use of fully human nanobody-producing mice has become an inevitable trend in the development of therapeutic nanobody drugs.

Common techniques for the preparation of animal models with large fragments of genes (more than hundreds of kilobases) being humanized include chromosome engineering, RMCE (recombinase-mediated cassette exchange) and single BAC transgenesis. Chromosome engineering has a high threshold with a development cycle of about 5 years, and relies on embryonic stem cells from the recipient species, limiting its use to species from which highly efficient embryonic stem cells can be isolated, such as mice. The size of the target gene fragment which is recombined into the genome through RMCE technology each time is about 200 kb, and as such, gene modification at the megabase scale requires at least 5 to 6 times of gene recombination on embryonic stem cells, and it will take about 5 years to construct a complete animal model. For the single BAC transgenesis, the transgene size is constrained by the size of BAC. Generally, the gene fragment transferred during a single transgenesis procedure is about 200 kb in size, and it is impossible to transfer genes at megabase scale.

In addition, a fully human nanobody-producing mouse generally exhibits an immune titer lower than a wild mouse, which also implies less diversity in the screened antibody sequences. Therefore, how to improve the immune titer of a fully human nanobody-producing mouse and how to increase the diversity of antibodies produced therein are technical problems that need to be urgently addressed in the field is.

The information disclosed in the Background Art is only intended to facilitate understanding of the general background of the present disclosure and should not be taken as an acknowledgment or an imply in any form that the information constitutes the prior art that is already known to one of ordinary skill in the art.

### Summary of the Invention

Purpose of Invention: In view of the above problems present in the prior art, the present invention aims to provide a method for preparing a genetically modified non-human mammal capable of efficiently producing a humanized antibody, including a whole antibody, a single heavy chain antibody, and a nanobody, a method for preparing a humanized antibody that binds specifically to an antigen by using the non-human mammal produced by the above method, and a method for acquiring a biological sample.

By using the MBGE delivery system, the method for preparing a genetically modified non-human mammal according to the invention can prepare a fully human nanobody-producing mouse with megabase-scale humanized genome fragments within a short period of time (e.g., within six months). Moreover, the efficiency of obtaining positive mice via a single injection is as high as about 15%, which makes it easy to prepare humanized mice with different antibody diversities (e.g., using a variety of strains of mice to increase nanobody sequence diversity). In addition, the prepared non-human mammal has a higher immune titer, allowing for efficient production of a humanized antibody, including a whole antibody, a single heavy chain antibody, and a nanobody. Technical Solutions: In order to achieve the purpose of the invention, the technical solutions are provided herein as follows:
In a first aspect, provided herein is a genetically modified non-human mammal, which comprises a disruption in an endogenous heavy chain immunoglobulin gene loci therein.

In some preferred embodiments, the non-human mammal is a mouse, and the disruption in the endogenous heavy chain immunoglobulin gene loci includes deletions of the following gene fragments:
a CH1 fragment of mouse antibody gene heavy chain IgHM, mouse antibody gene light chain Igxc, and a fragment between IgLc2 and IgLc1 of mouse antibody gene light chain Igλ.

In other preferred embodiments, the non-human mammal is a mouse, and the disruption in the endogenous heavy chain immunoglobulin gene loci includes deletions of the following gene fragments:
a fragment between IgHM and IgHA-CH1 of mouse antibody gene heavy chain, mouse antibody gene light chain Igxc, and a fragment between IgLc2 and IgLc1 of mouse antibody gene light chain Igλ.

The genetically modified non-human mammal as described above can be used as a background animal to prepare a non-human mammal capable of producing a human antibody by introducing appropriate human antibody genes thereinto.

In a second aspect, provided herein is a method for preparing the genetically modified non-human mammal according to the first aspect, which comprises the following steps:
bringing about a disruption into an endogenous heavy chain immunoglobulin gene loci of a non-human mammal.

Preferably, the non-human mammal is a mouse.

In some preferred embodiments, the disruption in the endogenous heavy chain immunoglobulin gene loci includes deletions of the following gene fragments:
a CH1 fragment of mouse antibody gene heavy chain IgHM, mouse antibody gene light chain Igxc, and a fragment between IgLc2 and IgLc1 of mouse antibody gene light chain Igλ.

Further preferably, the deletions of the gene fragments are performed through CRISPR-Cas9 gene editing technique;
wherein the sgRNA for deleting the CH1 fragment of mouse antibody gene heavy chain IgHM comprises a sgRNA as shown in SEQ ID NO: 1 and a sgRNA as shown in SEQ ID NO: 2; and/or, the sgRNA for deleting the mouse antibody gene light chain Igκc comprises a sgRNA as shown in SEQ ID NO: 3; and/or, the sgRNA for deleting the fragment between IgLc2 and IgLc1 of mouse antibody gene light chain Igλ, comprises a sgRNA as shown in SEQ ID NO: 4 and a sgRNA as shown in SEQ ID NO: 5.

In other preferred specific embodiments, the disruption in the endogenous heavy chain immunoglobulin gene loci includes deletions of the following gene fragments:
a fragment between IgHM and IgHA-CH1 of mouse antibody gene heavy chain, mouse antibody gene light chain Igxc, and a fragment between IgLc2 and IgLc1 of mouse antibody gene light chain Igλ.

Further preferably, the deletions of the gene fragments are performed through CRISPR-Cas9 gene editing technique;
wherein the sgRNA for deleting the fragment between IgHM and IgHA-CH1 of mouse antibody gene heavy chain comprises a sgRNA as shown in SEQ ID NO: 1 and a sgRNA as shown in SEQ ID NO: 6; and/or, the sgRNA for deleting the mouse antibody gene light chain Igκc comprises a sgRNA as shown in SEQ ID NO: 3; and/or, the sgRNA for deleting a fragment between IgLc2 and IgLc1 of mouse antibody gene light chain Igλ, comprises a sgRNA as shown in SEQ ID NO: 4 and a sgRNA as shown in SEQ ID NO: 5.

In a third aspect, provided herein is a genetically modified non-human mammal, wherein the non-human mammal comprises a disruption in an endogenous heavy chain immunoglobulin gene loci, and the endogenous heavy chain immunoglobulin gene loci comprises a human IGHV gene, a human IGHD gene, a human IGHJ gene as well as an endogenous IgHG2c gene, IgHE gene, IgHA gene, and LCR region of the non-human mammal.

In some preferred embodiments, the non-human mammal is a mouse, and the disruption in the endogenous heavy chain immunoglobulin gene loci includes deletions of the following gene fragments:
a CH1 fragment of mouse antibody gene heavy chain IgHM, mouse antibody gene light chain Igxc, and a fragment between IgLc2 and IgLc1 of mouse antibody gene light chain Igλ.

In other preferred embodiments, the non-human mammal is a mouse, and the disruption in the endogenous heavy chain immunoglobulin gene loci includes deletions of the following gene fragments:
a fragment between IgHM and IgHA-CH1 of mouse antibody gene heavy chain, mouse antibody gene light chain Igxc, and a fragment between IgLc2 and IgLc1 of mouse antibody gene light chain Igλ.

In feasible embodiments, the human IGHV gene includes at least the following 18 human IGHV genes:
hIGHV4-59, hIGHV3-53, hIGHV5-51, hIGHV3-49, hIGHV3-48, hIGHV1-46, hIGHV3-43, hIGHV4-39, hIGHV3-33, hIGHV4-30-2, hIGHV1-24, hIGHV3-23, hIGHV3-21, hIGHV3-11, hIGHV3-7, hIGHV4-4, hIGHV1-2, and hIGHV6-1;
preferably, the human IGHV gene further includes one or several or all of the human IGHV genes selected from: hIGHV1-58, hIGHV1-45, hIGHV3-35, hIGHV4-28, hIGHV2-26, hIGHV3-20, hIGHV1-18, hIGHV3-15, hIGHV3-13, hIGHV5-10-1, hIGHV3-64D, hIGHV2-5, hIGHV7-4-1, and hIGHV1-3;
further preferably, the human IGHV gene further includes one or several or all of the human IGHV genes selected from: hIGHV3-60, hIGHV3-57, hIGHV7-56, hIGHV4-55, hIGHV3-54, hIGHV3-52, hIGHV3-50, hIGHV3-47, hIGHV3-42, hIGHV3-41, hIGHV3-38, hIGHV3-37, hIGHV3-36, hIGHV7-34-1, hIGHV4-34, hIGHV3-33-2, hIGHV3-32, hIGHV3-30-2, hIGHV3-30, hIGHV3-29, hIGHV7-27, hIGHV3-25, hIGHV3-22, hIGHV3-19, hIGHV1-17, hIGHV3-16, hIGHV1-14, hIGHV1-12, and hIGHV3-6.

In feasible embodiments, the human IGHD gene includes at least the following 15 human IGHD genes:
IGHD1-1, IGHD3-3, IGHD6-6, IGHD1-7, IGHD3-10, IGHD6-13, IGHD1-14, IGHD2-15, IGHD3-16, IGHD5-18, IGHD6-19, IGHD1-20, IGHD3-22, IGHD1-26, and IGHD7-27;
preferably, the human IGHD gene further includes one or several or all of the human IGHD genes selected from: IGHD2-2, IGHD2-8, IGHD3-9, IGHD3-10, IGHD4-11, IGHD5-12, IGHD3-16, IGHD4-17, IGHD2-21, IGHD4-23, IGHD5-24, and IGHD6-25;
further preferably, the human IGHD gene further includes one or several or all of the human IGHD genes selected from: IGHD3-3, IGHD3-10, IGHD3-16, and IGHD5-18.

In feasible embodiments, the human IGHJ gene includes all the human IGHJ genes; and/or, the endogenous IgHG2c gene of the non-human mammal is the complete endogenous IgHG2c gene, or the endogenous IgHG2c gene fragment without the CH1 domain. When the endogenous IgHG2c gene of the non-human mammal is the complete endogenous IgHG2c gene, after antigen immunization, a humanized whole antibody is produced; when the endogenous IgHG2c gene of the non-human mammal is the endogenous IgHG2c gene fragment without the CH1 domain, after antigen immunization, a humanized single heavy chain antibody is produced.

Preferably, the human IGHV genes, the human IGHD genes, and the human IGHJ genes are operatively linked to each other and subjected to VDJ-rearrangement; further preferably, the operatively linked and/or VDJ-rearranged human IGHV genes, human IGHD genes, and human IGHJ genes, are operatively linked to the endogenous IgHG2c gene, IgHE gene, IgHA gene, and LCR region of the non-human mammal;
preferably, there is a Switch region of the endogenous IgHM of the non-human mammal between the human IGHJ gene and the endogenous IgHG2c gene of the non-human mammal.

Most preferably, the endogenous heavy chain immunoglobulin gene loci of the non-human mammal comprises all the genes shown in Table 13.

In a fourth aspect, provided herein is a method for preparing the genetically modified non-human mammal according to the third aspect, which comprises the following steps:
(1) bringing about a disruption into an endogenous heavy chain immunoglobulin gene loci of a non-human mammal;
(2) introducing a human IGHV gene, a human IGHD gene, a human IGHJ gene as well as an endogenous IgHG2c gene, IgHE gene, IgHA gene, and LCR region of the non-human mammal into the non-human mammal obtained in step (1).

In feasible embodiments, the non-human mammal is a mouse, and the disruption in the endogenous heavy chain immunoglobulin gene loci as described in step (1) includes deletions of the following gene fragments:
a CH1 fragment of mouse antibody gene heavy chain IgHM, mouse antibody gene light chain Igxc, and a fragment between IgLc2 and IgLc1 of mouse antibody gene light chain Igλ;
preferably, the deletions of the gene fragments are performed through gene editing techniques such as CRISPR-Cas9 technique;
further preferably, the sgRNA for deleting the CH1 fragment of mouse antibody gene heavy chain IgHM comprises a sgRNA as shown in SEQ ID NO: 1 and a sgRNA as shown in SEQ ID NO: 2; and/or, the sgRNA for deleting the mouse antibody gene light chain Igκc comprises a sgRNA as shown in SEQ ID NO: 3; and/or, the sgRNA for deleting the fragment between IgLc2 and IgLc1 of mouse antibody gene light chain Igλ comprises a sgRNA as shown in SEQ ID NO: 4 and a sgRNA as shown in SEQ ID NO: 5.

In some other feasible embodiments, the non-human mammal is a mouse, and the disruption in the endogenous heavy chain immunoglobulin gene loci as described in step (1) includes deletions of the following gene fragments:
a fragment between IgHM and IgHA-CH1 of mouse antibody gene heavy chain, mouse antibody gene light chain Igxc, and a fragment between IgLc2 and IgLc1 of mouse antibody gene light chain Igλ;
preferably, the deletions of the gene fragments are performed through gene editing techniques such as CRISPR-Cas9 technique;
further preferably, the sgRNA for deleting the fragment between IgHM and IgHA-CH1 of mouse antibody gene heavy chain comprises a sgRNA as shown in SEQ ID NO: 1 and a sgRNA as shown in SEQ ID NO: 6; and/or, the sgRNA for deleting the mouse antibody gene light chain Igκc comprises a sgRNA as shown in SEQ ID NO: 3; and/or the sgRNA for deleting a fragment between IgLc2 and IgLc1 of mouse antibody gene light chain Igλ comprises a sgRNA as shown in SEQ ID NO: 4 and a sgRNA as shown in SEQ ID NO: 5.

In some specific embodiments, the non-human mammal is a mouse, and in step (2), the human IGHV gene includes at least the following 18 human IGHV genes:
hIGHV4-59, hIGHV3-53, hIGHV5-51, hIGHV3-49, hIGHV3-48, hIGHV1-46, hIGHV3-43, hIGHV4-39, hIGHV3-33, hIGHV4-30-2, hIGHV1-24, hIGHV3-23, hIGHV3-21, hIGHV3-11, hIGHV3-7, hIGHV4-4, hIGHV1-2, and hIGHV6-1; optionally, the human IGHV gene further includes one or several or all of the human IGHV genes selected from: hIGHV1-58, hIGHV1-45, hIGHV3-35, hIGHV4-28, hIGHV2-26, hIGHV3-20, hIGHV1-18, hIGHV3-15, hIGHV3-13, hIGHV5-10-1, hIGHV3-64D, hIGHV2-5, hIGHV7-4-1, and hIGHV1-3; and further optionally,
the human IGHV gene further includes one or several or all of the human IGHV genes selected from: hIGHV3-60, hIGHV3-57, hIGHV7-56, hIGHV4-55, hIGHV3-54, hIGHV3-52, hIGHV3-50, hIGHV3-47, hIGHV3-42, hIGHV3-41, hIGHV3-38, hIGHV3-37, hIGHV3-36, hIGHV7-34-1, hIGHV4-34, hIGHV3-33-2, hIGHV3-32, hIGHV3-30-2, hIGHV3-30, hIGHV3-29, hIGHV7-27, hIGHV3-25, hIGHV3-22, hIGHV3-19, hIGHV1-17, hIGHV3-16, hIGHV1-14, hIGHV1-12, and hIGHV3-6;
and/or, the human IGHD gene includes at least the following 15 human IGHD genes:
IGHD1-1, IGHD3-3, IGHD6-6, IGHD1-7, IGHD3-10, IGHD6-13, IGHD1-14, IGHD2-15, IGHD3-16, IGHD5-18, IGHD6-19, IGHD1-20, IGHD3-22, IGHD1-26, and IGHD7-27; optionally, the human IGHD gene further includes one or several or all of the human IGHD genes selected from: IGHD2-2, IGHD2-8, IGHD3-9, IGHD3-10, IGHD4-11, IGHD5-12, IGHD3-16, IGHD4-17, IGHD2-21, IGHD4-23, IGHD5-24, and IGHD6-25; and further optionally, the human IGHD gene further includes one or several or all of the human IGHD genes selected from: IGHD3-3, IGHD3-10, IGHD3-16, and IGHD5-18;
and/or, the human IGHJ gene includes all the human IGHJ genes;
and/or, the endogenous IgHG2c gene of the non-human mammal is the complete endogenous IgHG2c gene, or the endogenous IgHG2c gene fragment without the CH1 domain. When the endogenous IgHG2c gene of the non-human mammal is the complete endogenous IgHG2c gene, after antigen immunization, a humanized whole antibody is produced; when the endogenous IgHG2c gene of the non-human mammal is the endogenous IgHG2c gene fragment without the CH1 domain, after antigen immunization, a humanized single heavy chain antibody is produced.

In a preferred embodiment of the above specific embodiments, in step (2), the human IGHV gene, the human IGHD gene, the human IGHJ gene as well as the endogenous IgHG2c gene, IgHE gene, IgHA gene, and LCR region of the non-human mammal are introduced by the following procedure:
introducing the following materials into the mouse obtained in step (1):
(I) n BAC clones that comprise all of the human IGHV genes, the human IGHD genes, and the human IGHJ genes, in total; wherein n is an integer between 3 and 8, preferably between 4 and 7, most preferably, n is 4; each of the n BAC clones has a 5 kb to 50 kb of gene fragment at the beginning or the end thereof, and for every two adjacent BAC clones, the 5 kb to 50 kb of gene fragment at the end of one BAC clone is homologous to that at the beginning of the other BAC clone, so that they can be interconnected with each other through the homologous gene sequences (e.g., a 10 kb of gene fragment at the end of the n BAC clone is homologous to that at the beginning of the n+1 BAC clone); and
(II) one or more BAC clones comprising the endogenous IgHG2c gene, IgHE gene, IgHA gene, and LCR region of the non-human mammal.

Further preferably, in step (2), the human IGHV gene, the human IGHD gene, the human IGHJ gene as well as the endogenous IgHG2c gene, IgHE gene, IgHA gene, and LCR region of the non-human mammal are introduced by the following procedure:
introducing the following 6 BAC clones into the mouse obtained in step (1):
   (i) 4 BAC clones containing all the human IGHV genes,
   (ii) 1 BAC clone containing all the human IGHD genes and all the human IGHJ genes, and
   (iii) 1 BAC clone containing the endogenous IgHG2c gene, IgHE gene, IgHA gene, and LCR region of the non-human mammal;
wherein for the BAC clones in (i), and for the BAC clones in (i) and (ii), each BAC clone has a 5 kb to 50 kb, preferably 5 kb to 20 kb, of gene fragment at the beginning or the end thereof, and for every two adjacent BAC clones, the 5 kb to 50 kb, preferably 5 kb to 20 kb, of gene fragment at the end of one BAC clone is homologous to that at the beginning of the other BAC clone, so that they can be interconnected with each other through the homologous gene sequences;
preferably, the genes contained in the 6 BAC clones are shown in Table 13.

In the non-human mammal prepared according to the above method, the human IGHV genes, the human IGHD genes, and the human IGHJ genes are operatively linked to each other and subjected to VDJ-rearrangement, and the operatively linked and/or VDJ-rearranged human IGHV genes, human IGHD genes, and human IGHJ genes, are operatively linked to the endogenous IgHG2c gene, IgHE gene, IgHA gene, and LCR region of the non-human mammal;
and/or, there is a Switch region of the endogenous IgHM of the non-human mammal between the human IGHJ gene and the endogenous IgHG2c gene of the non-human mammal.

In a fifth aspect, provided herein a method for preparing a humanized whole antibody or a humanized single heavy chain antibody or a humanized nanobody that specifically binds to an antigen, comprising:
(1) the genetically modified non-human mammal according to the third aspect or the genetically modified non-human mammal prepared by the method according to the fourth aspect is exposed to an antigen;
(2) B cells are collected from the non-human mammal obtained in step (1), RNA is extracted and then reverse transcribed into cDNA, and antibody gene fragments are amplified using the cDNA as the template, and then are cloned into a phage display vector;
(3) the phage display vector obtained in step (2) is used to express the target antibody; specifically, phages expressing the target antibody are enriched by panning, and then are induced to express the target antibody, i.e. a humanized whole antibody or a humanized single heavy chain antibody; and optionally, (4) the variable region fragment of the obtained single heavy chain antibody is cloned to obtain a humanized nanobody.

In a sixth aspect, provided herein is a method for preparing a humanized single heavy chain antibody or a humanized nanobody that specifically binds to an antigen, comprising:
(1) the genetically modified non-human mammal according to the third aspect or the genetically modified non-human mammal prepared by the method according to the fourth aspect is exposed to an antigen, and then B cells are collected;
(2) nucleic acids encoding the immunoglobulin heavy chain variable region and optional the light chain variable region in the B cells collected in step (1) are sequenced to obtain the nucleic acid sequences of a heavy chain variable region and a light chain variable region of a humanized monoclonal antibody or the nucleic acid sequence of a heavy chain variable region of a humanized nanobody,
(3) according to the sequences obtained in step (2), a humanized whole antibody or a humanized single heavy chain antibody that specifically binds to the antigen is obtained by expression; and optionally, (4) the variable region fragment of the obtained single heavy chain antibody is cloned to obtain a humanized nanobody.

In a seventh aspect, provided herein is a method for acquiring a biological sample, comprising:
(1) the genetically modified non-human mammal according to the third aspect or the genetically modified non-human mammal prepared by the method according to the fourth aspect is exposed to an antigen;
(2) the biological sample is collected from the non-human mammal.

Preferably, the biological sample is selected from spleen tissues, splenocytes, or B cells.

In an eighth aspect, provided herein is a biological sample which is obtained by the method according to the seventh aspect.

In a ninth aspect, provided herein is a sgRNA composition, which comprises: sgRNA for deleting the CH1 fragment of mouse antibody gene heavy chain IgHM; sgRNA for deleting the mouse antibody gene light chain Igκc; and sgRNA for deleting a fragment between IgLc2 and IgLc1 of mouse antibody gene light chain Igλ.

Preferably, the sgRNA for deleting the CH1 fragment of mouse antibody gene heavy chain IgHM comprises a sgRNA as shown in SEQ ID NO: 1 and a sgRNA as shown in SEQ ID NO: 2; preferably, the sgRNA for deleting the mouse antibody gene light chain Igκc comprises a sgRNA as shown in SEQ ID NO: 3;
preferably, the sgRNA for deleting the fragment between IgLc2 and IgLc1 of mouse antibody gene light chain Igλ, comprises a sgRNA as shown in SEQ ID NO: 4 and a sgRNA as shown in SEQ ID NO: 5.

In a tenth aspect, provided herein is a sgRNA composition, which comprises: sgRNA for deleting a fragment between IgHM and IgHA-CH1 of mouse antibody gene heavy chain; sgRNA for deleting the mouse antibody gene light chain Igκc; and sgRNA for deleting a fragment between IgLc2 and IgLc1 of mouse antibody gene light chain Igλ.

Preferably, the sgRNA for deleting the fragment between IgHM and IgHA-CH1 of mouse antibody gene heavy chain comprises a sgRNA as shown in SEQ ID NO: 1 and a sgRNA as shown in SEQ ID NO: 6;
preferably, the sgRNA for deleting the mouse antibody gene light chain Igκc comprises a sgRNA as shown in SEQ ID NO: 3;
preferably, the sgRNA for deleting the fragment between IgLc2 and IgLc1 of mouse antibody gene light chain Igλ, comprises a sgRNA as shown in SEQ ID NO: 4 and a sgRNA as shown in SEQ ID NO: 5.

In an eleventh aspect, provided herein is a CRISPR-Cas9 gene editing system, comprising the sgRNA composition according to the ninth or tenth aspect and Cas9 protein.

In a twelfth aspect, provided herein is a gene knockout vector containing DNA sequence(s) encoding the sgRNA(s) in the sgRNA composition according to the ninth or tenth aspect.

In a thirteenth aspect, provided herein is a mouse embryonic stem cell, comprising the gene knockout vector according to the twelfth aspect.

Beneficial effects: The genetically modified non-human mammals prepared according to the method of the present invention exhibit a higher immune titer, are capable of efficiently producing a humanized single heavy chain antibody or nanobody after antigen immunization, and thereby can serve as an efficient platform for producing a humanized whole antibody or single heavy chain antibody or nanobody. Furthermore, the method for preparing the non-human mammal of the present invention has a relatively high efficiency (about 15%) in obtaining positive animals, thereby making it easy to prepare humanized mice with diverse antibodies, which allows for the realization of nanobody sequence diversity by a variety of strains of mice.

### Brief Description of the Drawings

One or more examples are exemplified by the pictures in the accompanying drawings that correspond thereto and are not intended to be limiting of the embodiments. As used herein, the word "exemplary" means "serving as an example, embodiment, or illustrative". Any embodiment described herein as "exemplary" is not necessarily to be construed as superior or better than other embodiments.

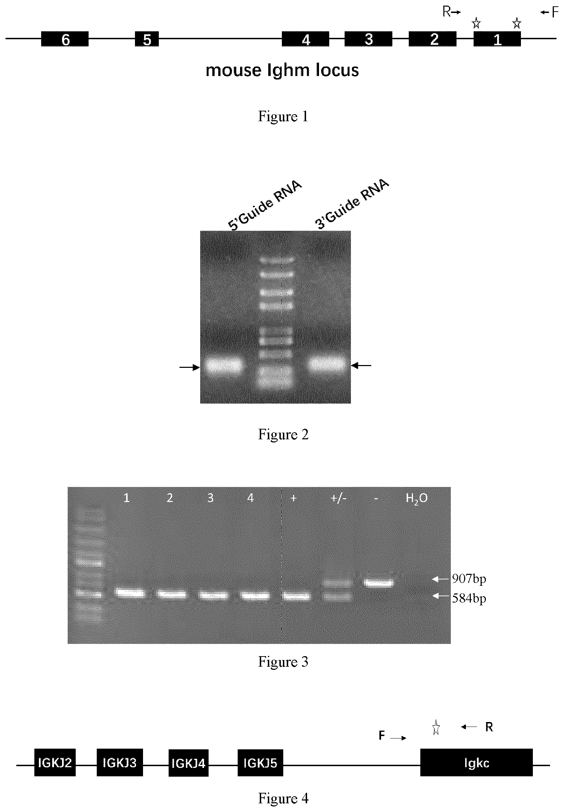

Figure 1 shows a schematic diagram of the targets of the sgRNAs for deleting the CH1 region of mouse IgHM gene as recited in Example 1, wherein the five-star symbols denote the locations of upstream and downstream targets, respectively.
Figure 2 shows an image of agarose gel electrophoresis of transcription products of the sgRNAs for deleting the CH1 region of mouse IgHM gene as recited in Example 1.
Figure 3 shows an image of agarose gel electrophoresis of PCR products for detecting gene deletions in the F2 generation mice and their offspring homozygous mice as recited in Example 1, wherein, the numbers 1 to 4 represent the clone numbers of the tested mice, "+" represents a homozygous positive control, " +/-" represents a heterozygous control, "-" represents a wild-type mouse control, and "H₂O" represents a water control.
Figure 4 shows a schematic diagram of the target of the sgRNA for deleting mouse antibody gene light chain Igxc as recited in Example 2.

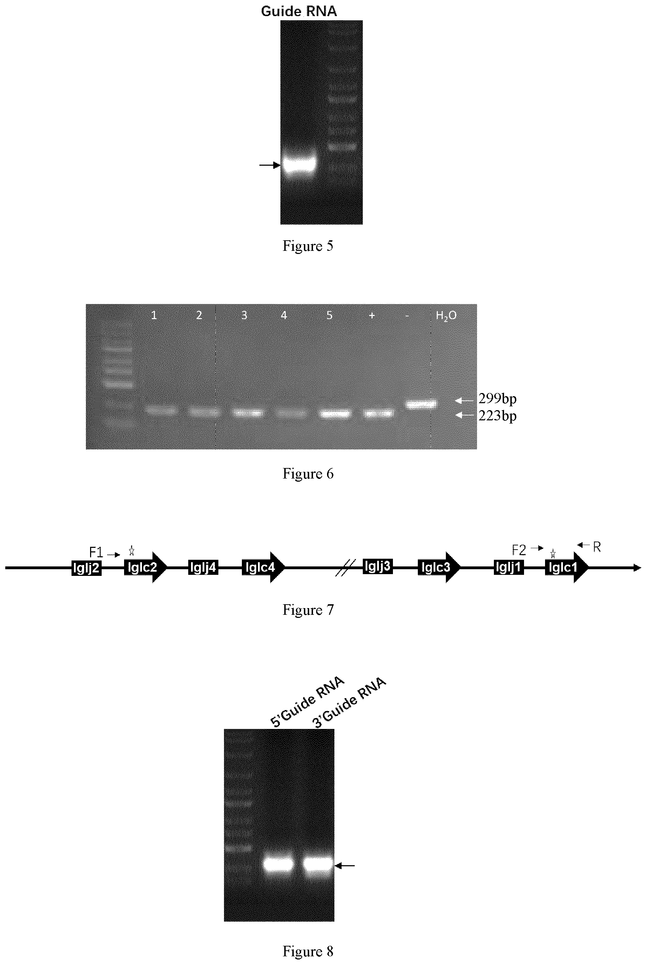

Figure 5 shows an image of agarose gel electrophoresis of the transcription product of the sgRNA for deleting mouse antibody gene light chain Igxc as recited in Example 2.
Figure 6 shows an image of agarose gel electrophoresis of PCR products for detecting gene deletions in the F2 generation mice and their offspring homozygous mice as recited in Example 2, wherein, the numbers 1 to 5 represent the clone numbers of the tested mice, "+" represents a homozygous positive control, " +/-" represents a heterozygous control, "-" represents a wild-type mouse control, and "H₂O" represents a water control.
Figure 7 shows a schematic diagram of the targets of the sgRNAs for deleting a fragment between IgLc2 and IgLc1 of mouse antibody gene light chain Igλ, as recited in Example 3, wherein the five-star symbols denote the locations of upstream and downstream targets, respectively.
Figure 8 shows an image of agarose gel electrophoresis of transcription products of the sgRNAs for deleting the fragment between IgLc2 and IgLc1 of mouse antibody gene light chain Igλ, as recited in Example 3, wherein the expression "5' Guide RNA" denotes GuideRNA at the position Iglc2, and the expression "3' Guide RNA" denotes GuideRNA at the position Iglc1.

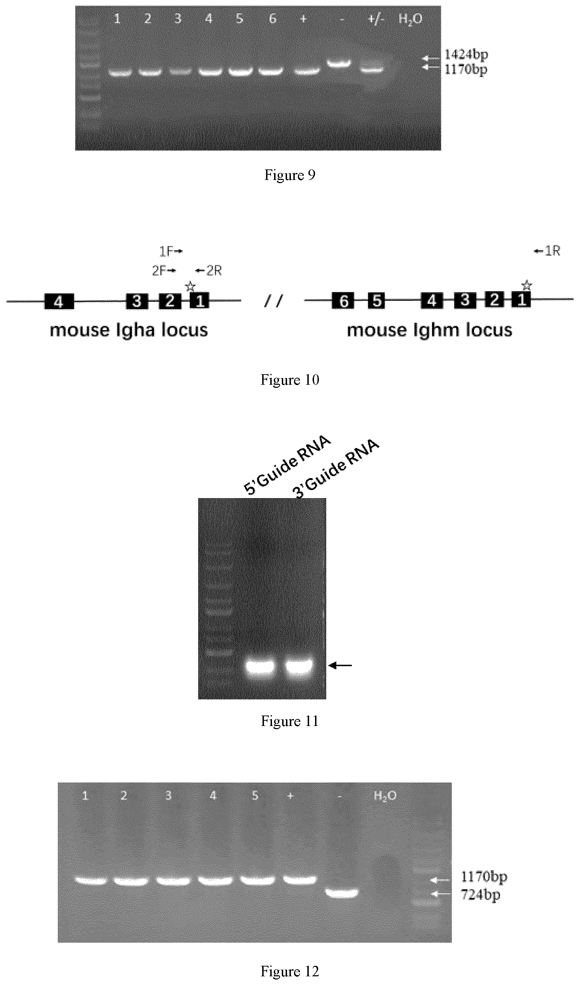

Figure 9 shows an image of agarose gel electrophoresis of PCR products for detecting gene deletions in the F2 generation mice and their offspring homozygous mice as recited in Example 3, wherein, the numbers 1 to 6 represent the clone numbers of the tested mice, "+" represents a homozygous positive control, " +/-" represents a heterozygous control, "-" represents a wild-type mouse control, and "H₂O" represents a water control.
Figure 10 shows a schematic diagram of the targets of the sgRNAs for deleting a fragment between IgHM and IgHA-CH1 of mouse antibody gene heavy chain as recited in Example 4, wherein the five-star symbols denote the locations of upstream and downstream targets, respectively.
Figure 11 shows an image of agarose gel electrophoresis of the transcription products of sgRNAs for deleting the fragment between IgHM and IgHA-CH1 of mouse antibody gene heavy chain as recited in Example 4, wherein the expression "5' Guide RNA" denotes GuideRNA at the position IgM, and the expression "3' Guide RNA" denotes GuideRNA at the position IgHA.
Figure 12 shows an image of agarose gel electrophoresis of PCR products for detecting gene deletions in the F2 generation mice and their offspring homozygous mice as recited in Example 4, wherein, the numbers 1 to 5 represent the clone numbers of the tested mice, "+" represents a homozygous positive control, " +/-" represents a heterozygous control, "-" represents a wild-type mouse control, and "H₂O" represents a water control.

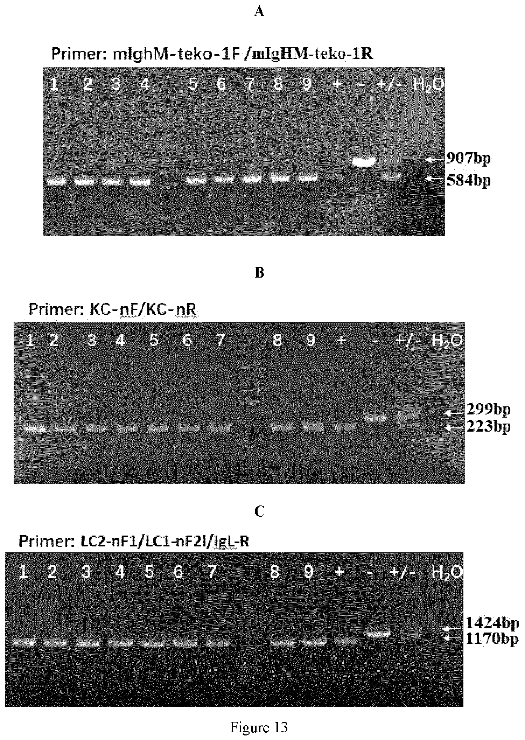

Figure 13 shows an image of agarose gel electrophoresis of PCR products for detecting deletions of IgM (A), IgK (B), and IgL (C) genes in the gene knockout mouse as recited in Example 5, wherein, the numbers 1 to 9 represent the clone numbers of the tested mice, "+" represents a homozygous positive control, " +/-" represents a heterozygous control, "-" represents a wild-type mouse control, and "H₂O" represents a water control.

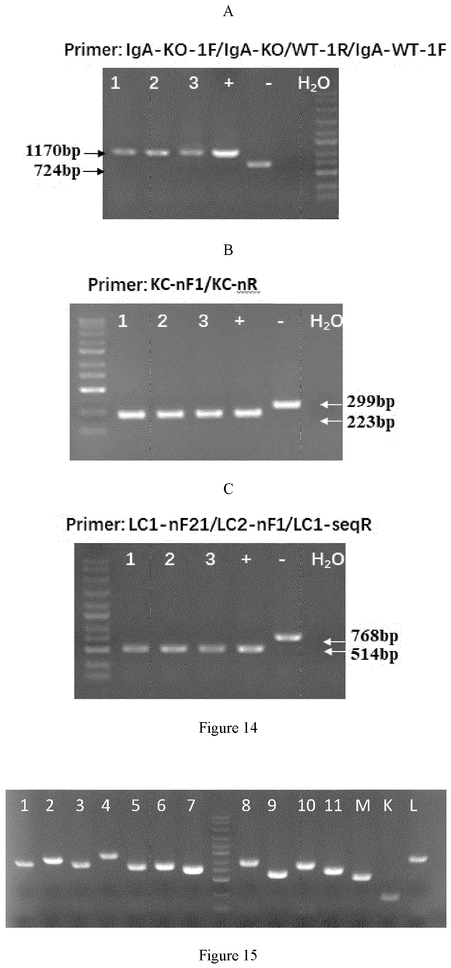

Figure 14 shows an image of agarose gel electrophoresis of PCR products for detecting deletions of IgA (A), IgK (B), and IgL (C) genes in the gene knockout mouse as recited in Example 6, wherein, the numbers 1 to 3 represent the clone numbers of the tested mice, "+" represents a homozygous positive control, "-" represents a wild-type mouse control, and "H₂O" represents a water control.
Figure 15 shows an image of agarose gel electrophoresis of PCR products for identifying the genotype of the F2 generation IgM-KL-hIgHD mice as recited in Example 7, wherein the numbers 1 to 11 correspond to the primer numbers shown in Table 11, "M" corresponds to the PCR band for identifying IgM, "K" corresponds to the PCR band for identifying IgK, and "L" corresponds to the PCR band for identifying IgL.

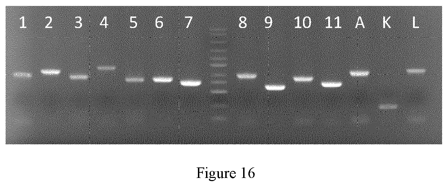

Figure 16 an image of agarose gel electrophoresis of PCR products for identifying the genotype of the F2 generation IgA-KL-hIgHD mice as recited in Example 8, wherein the numbers 1 to 11 correspond to the primer numbers shown in Table 11, "A" corresponds to the PCR band for identifying IgA, "K" corresponds to the PCR band for identifying IgK, and "L" corresponds to the PCR band for identifying IgL.

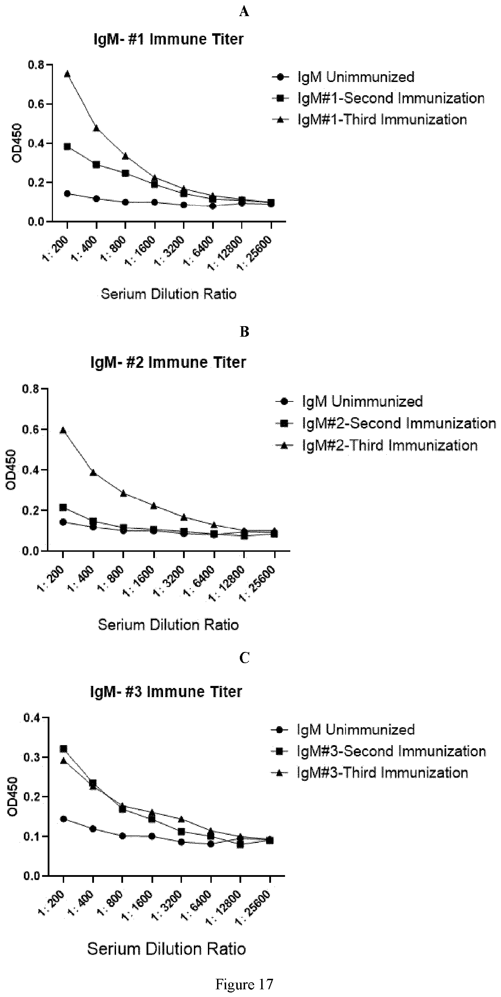

FIGS. 17A-C show plots of the immune titer detection results of three IgM homozygous mice immunized with OVA antigen as recited in Example 9.

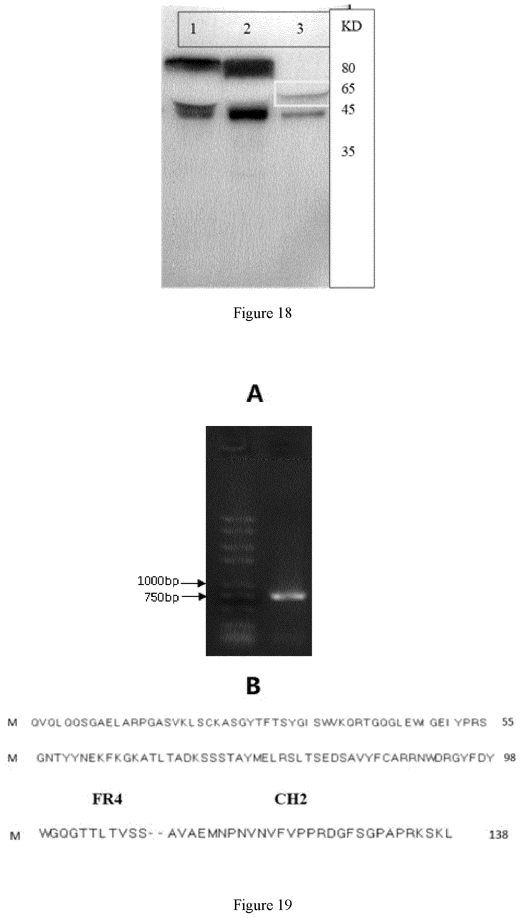

Figure 18 shows an image of Western Blot assay results of the serum from IgM gene knockout mice immunized with OVA antigen under reducing conditions as recited in Example 9, wherein lane 1 represents an immunized C57BL/6 wild-type mouse serum sample, lane 2 represents an immunized IgM CH1 ko heterozygous mouse serum sample, and lane 3 represents an immunized IgM CH1 ko homozygous mouse serum sample, and the above band in each lane represents IgM heavy chain.
Figure 19 shows an image of RT-PCR detection results of splenocytes from IgM gene knockout mice immunized with OVA antigen as recited in Example 9, wherein panel A shows the colony PCR results of the selected clones, and panel B shows partial sequencing results of the selected clones.

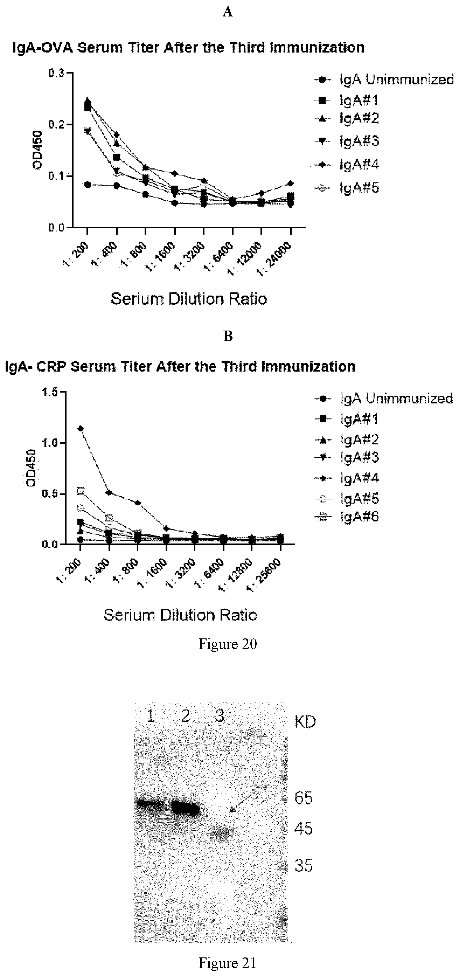

Figure 20 shows plots of the immune titer detection results of IgA homozygous mice immunized with OVA (A) and CRP (B) antigens, respectively, as recited in Example 10.
Figure 21 shows an image of Western blot assay results of the serum from IgA homozygous mice immunized with CRP antigen under reducing conditions as recited in Example 10, wherein lane 1 and lane 2 represent the immunized C57BL/6 mouse serum samples, respectively, and lane 3 represents the immunized IgA homozygous mouse serum sample.

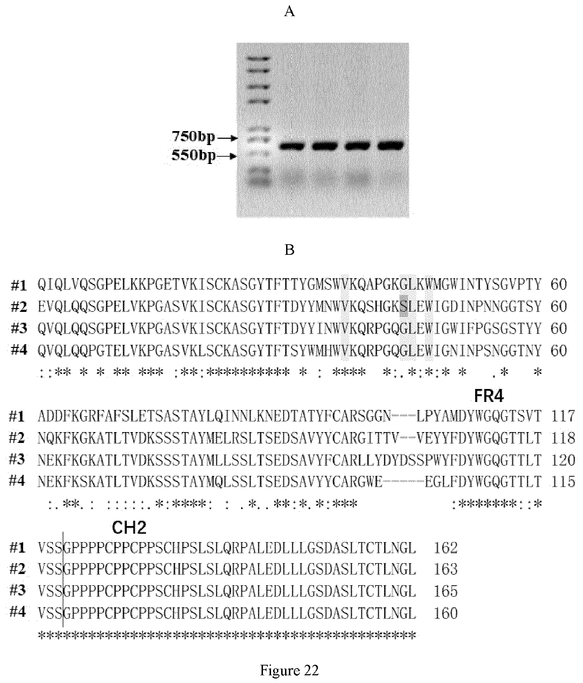

Figure 22 shows an image of RT-PCR detection results of splenocytes from IgA homozygous mice immunized with CRP antigen as recited in Example 10, wherein panel A shows the colony PCR results of the selected clones, and panel B shows partial sequencing results of the selected clones.

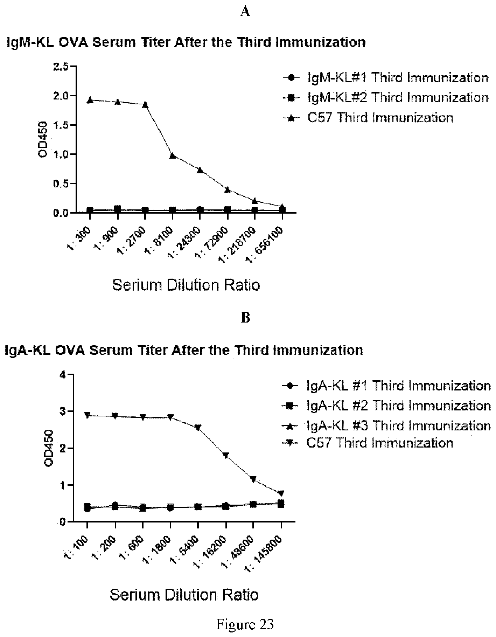

Figure 23 shows plots of the immune titer detection results of IgM-KL (A) and IgA-KL (B) homozygous mice immunized with OVA antigen as recited in Example 11.

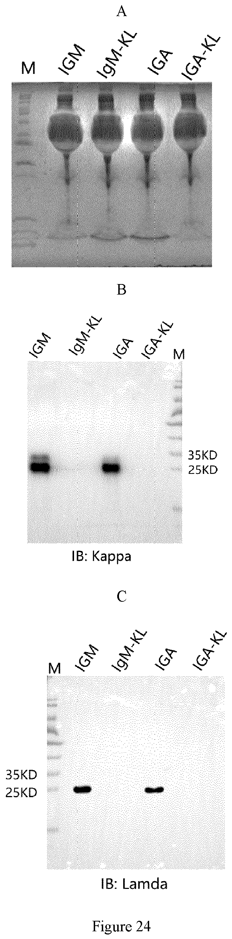

FIGS. 24A-C show images of Coomassie Brilliant Blue staining results (A) of sera, Western blot results of Kappa chain in sera (B), and Western blot results of Lamda chain in sera (C), where the sera are derived from IgM, IgM-KL, IgA, and IgA-KL mice immunized with OVA antigen, as recited in Example 11.

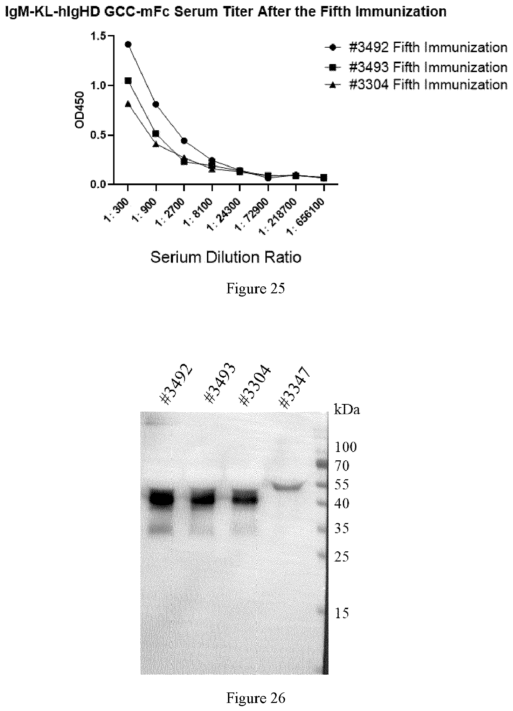

FIGS. 25 show a plot of the immune titer detection results of IgM-KL-hIgHD homozygous mice immunized with GCC-mFc protein, as recited in Example 12.
Figure 26 shows an image of Western blot assay results of serum samples from IgM-KL-hIgHD homozygous mice immunized with GCC-mFc protein under reducing conditions as recited in Example 12, wherein the lanes labelled #3492, #3493, #3304 represent serum samples from IgM-KL-hIgHD homozygous mice immunized with GCC-mFc antigen, and the lane labelled #3347 represents the serum sample from unimmunized C57BL/6 mice.

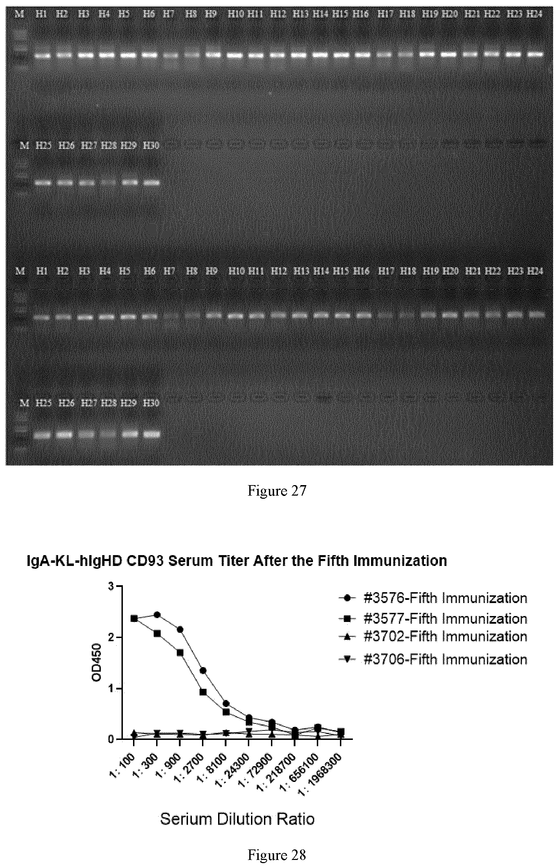

Figure 27 shows an image of RT-PCR product detection results of splenocytes from IgM-KL-hIgHD homozygous mice as recited in Example 12, wherein rows 1 and 2 represent the PCR products of unimmunized IgM-KL-hIgHD mouse samples, and rows 3 and 4 represent the PCR products of IgM-KL-hIgHD samples immunized with GCC-mFc.
Figure 28 shows a plot of the immune titer detection results of IgA-KL-hIgHD homozygous mice immunized with CD93 protein, as recited in Example 13.

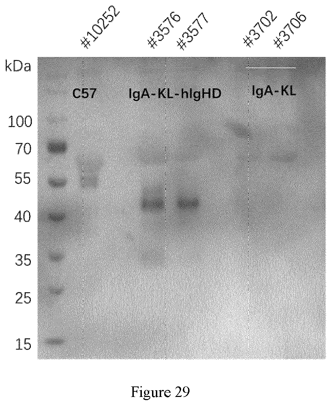

Figure 29 shows an image of Western blot assay results of serum samples from IgA-KL-hIgHD homozygous mice immunized with CD93 protein as recited in Example 13, wherein the lanes labeled #3756, #3577 represent serum samples of IgA-KL-hIgHD homozygous mice immunized with CD93 antigen, the lanes labeled #3702, #3706 represent serum samples of IgA-KL mice immunized with CD93 antigen, and the lane labelled #10252 represents the serum sample of unimmunized C57BL/6 mice.

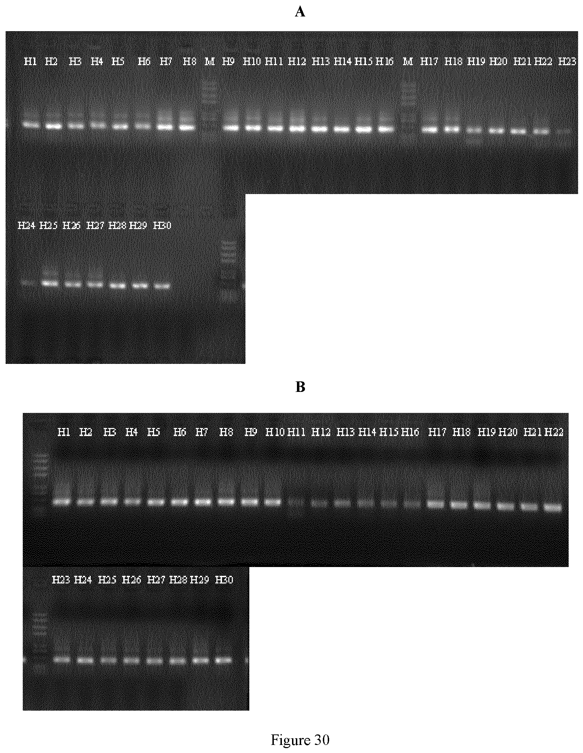

Figure 30 shows an image of RT-PCR product detection results of splenocytes from IgA-KL-hIgHD homozygous mice as recited in Example 14, wherein panel A represents the PCR product of unimmunized IgA-KL-hIgHD mouse sample, and panel B represents the PCR product of IgA-KL-hIgHD sample immunized with CD93 protein.

### Detailed Description of the Invention

In order to make the purpose, technical solutions and advantages of the embodiments of the invention clearer, the technical solutions in the embodiments of the invention will be described clearly and completely, obviously, the described embodiments are some of the embodiments of the present invention, but not all of them. Based on the embodiments of the present invention, all other embodiments obtained by those of ordinary skill in the art without creative work are within the scope of the present invention.

In addition, in order to better explain the present invention, a lot of specific details are given in the following embodiments. It will be understood by those skilled in the art that the present invention may be practiced without certain specific details. In some embodiments, materials, elements, methods, means, etc., well known to those skilled in the art, are not described in detail so as to highlight the spirit of the present invention.

Throughout the specification and claims, the term "comprising" or variations thereof, such as "including" or "containing", will be understood to include the stated components and not to exclude other elements or other components, unless expressly indicated otherwise.

In addition, in the following embodiments, the mice used were C57BL/6 mice purchased from Viton Lever, and the starting mice were aged 4 to 6 weeks and weighed about 18 g to 22 g.

**Example 1: Design of sgRNAs for deleting a CH1 region of mouse IgHM gene (hereinafter also referred to as "IgM gene"), and preparation and identification of gene knockout mice (hereinafter referred to as "IgM mice" or "IgM homozygous mice")**

To delete the CH1 region of mouse IgHM gene, two target sites were selected, one on the upstream of the first exon of mouse IgHM gene and the other on the downstream thereof, and one sgRNA was designed for each target site. Figure 1 shows a schematic diagram of the targets of the sgRNAs, wherein the five-star symbols denote the locations of upstream and downstream targets, respectively. The sequences of the designed two sgRNAs and their target sequences were shown in Table 1.

### 1. Construction of IgM mouse

### 1.1 Specific steps, including: sgRNA primer design, sgRNA in vitro transcription, mouse embryo injection, and positive mouse identification.

### 1.2 Experimental reagents:

MEGAshortscript^{™} Kit;
MEGAclear^{™} Kit;
Purification for Large Scale Transcription Reactions.
1.3 sgRNA primer design

**Table 1**

| **Mouse strain** | **sgRNA's targeting sequence (5'>3')** | **Designed sgRNA sequence (5'>3')** |
|---|---|---|
| IgM | GACTCTCTGAGGACCAGA GAGGG (SEQ ID NO: 7) | GACUCUCUGAGGACCAGAGAguuuaagagcuaugc uggaaacagcauagcaaguuuaaauaaggcuaguccguuaucaac uugaaaaaguggcaccgagucggugcuuuuuuu (SEQ ID NO: 1) |
| | GATCTGCATGTGCCCATTC CAGG (SEQ ID NO: 8) | GAUCUGCAUGUGCCCAUUCCguuuaagagcuaugc uggaaacagcauagcaaguuuaaauaaggcuaguccguuaucaac uugaaaaaguggcaccgagucggugcuuuuuuu(SEQ ID NO: 2) |

### 1.4 Preparation of sgRNA transcription template

### 1.4.1 Taq-mix PCR system was shown in Table 2, with each sample being amplified in 20 µL × 2 tubes.

**Table 2**

| | |
|---|---|
| ddH₂O | 8µL |
| Forward primer | 0.5µL |
| Reverse primer | 0.5µL |
| Template(~10ng/µL ) | 1µL |
| Taq mix | 10µL |
| Total Volume | 20µL |

### 1.4.2 Touchdown program was shown in Table 3.

**Table 3**

| | | |
|---|---|---|
| 94°C | 5min | 1 cycle |
| 94°C | 30s | 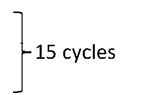 |
| 62°C-0.7°C/cycle | 30s | |
| 72°C | 15s | |
| 94°C | 30s | 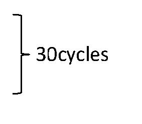 |
| 52°C | 30s | |
| 72°C | 15s | |
| 72°C | 5min | 1 cycle |

PCR products were recovered with a recovery kit and used for the subsequent experiment, that is, in vitro transcription.

### 1.5 In vitro transcription

### 1.5.1 A transcription system was shown in Table 4.

**Table 4**

| | |
|---|---|
| T7 10×Reaction Buffer | 2 µL |
| T7 ATP Solution (75 mM) | 2 µL |
| T7 CTP Solution (75 mM) | 2 µL |
| T7 GTP Solution (75 mM) | 2 µL |
| T7 UTP Solution (75 mM) | 2 µL |
| Template DNA | 8 µL |
| T7 Enzyme Mix | 2 µL |

The above transcription system was incubated in a 37 °C incubator for 18 h and recovered with a kit known as MEGAclear^{™} Kit Purification for Large Scale Transcription Reactions.

### 1.5.2 Agarose gel electrophoresis result of sgRNA transcription products was shown in Figure 2.

### 1.6 Implantation of cells containing the above sgRNAs and Cas9 protein into a host animal:

Mice were subjected to ovulation induction and then in vitro fertilization to obtain fertilized eggs, which were cultured; then, sgRNAs and Cas9 protein were mixed together and electroporated into the fertilized eggs of mice, or Cas9 protein (or Cas9 mRNA, commercially available) along with sgRNAs were injected into the fertilized eggs of mice via microinjection.

The resultant fertilized egg cells were implanted into surrogate female mice, thereby producing F0 generation chimeric mice. By extracting genomic DNA from the mouse tail, performing PCR amplification, and analyzing the PCR amplification products, F0 generation positive knockout mice were identified.

In the above PCR amplification, the used PCR primers mIghM-teko-1F and mIghM-teko-1R were designed at both terminals of the deleted sequence. The sequences of the primers were shown in Table 5.

**Table 5**

| **Primer** | **Sequence (5'>3')** |
|---|---|
| mIgHM-teko-1F | CTGAAGGGCCAGATCCACCTACTCT (SEQ ID NO: 9) |
| mIgHM-teko-1R | TTGGGGTGTGGATCCTTTGTTCTCG (SEQ ID NO: 10) |

Analyses of PCR amplification products included agarose gel electrophoresis and sequencing analyses so as to determine whether the target sequence had been deleted.

According to the sequencing results of the PCR products, it was determined that the deleted sequence was 323bp in length. Therefore, the PCR products amplified with the above primers could detect whether the deleted genes or the wild-type genes, depending on the size of the PCR product fragments, wherein for the wild-type genes and those genes with the target sequence deleted, the target bands amplified with the primers mIghM-teko-1F and mIghM-teko-1R were 907bp and 584bp in size, respectively (the results were not shown).

F0 generation chimeric mice with correct gene knockout were selected for the subsequent reproduction and identification.

### 1.7 Reproduction of heterozygous and homozygous gene knockout mice

F0 generation mice with target gene knockout were mated with wild-type mice to obtain F1 generation mice. By extracting genomic DNA from the mouse tail and performing PCR detection, stably heritable gene knockout positive F1 generation heterozygous mice were selected. The F1 generation heterozygous mice were then mated with each other to obtain gene knockout positive F2 generation homozygous mice, i.e., IgM knockout homozygous mice. The obtained F2 generation mice and their offspring homozygous mice were subjected to genotype identification in the same way as that in step 1.6 above. Meanwhile, the gene knockout homozygous mouse and heterozygous mouse identified by sequencing were used as a homozygous positive control (the lane labeled "+") and a heterozygous control (the lane labeled "+/-"), respectively. Furthermore, a wild-type mouse control (the lane labeled "-") and a negative control without DNA template (also called a "water control") were also set up. The results were shown in Figure 3.

As shown in Figure 3, IgM knockout homozygous mice were indeed obtained following the above procedure.

### Example 2: Design of sgRNA for deleting mouse antibody gene light chain Igκc (hereinafter referred to as "IgK gene"), and preparation and identification of gene knockout mice (hereinafter referred to as " IgK mice" or " IgK homozygous mice")

To knock out the mouse Igxc gene, one target on the exon of the mouse Igxc gene was selected, and one sgRNA was designed for this target. The target schematic diagram of sgRNA was shown in Figure 4, and the designed sgRNA sequence and its target sequence were shown in Table 3.

### 2. Construction of IgK mice

2.1 Specific steps, including: sgRNA primer design, sgRNA in vitro transcription, mouse embryo injection, and positive mouse identification.

### 2.2 Experimental reagents:

MEGAshortscript^{™} Kit;
MEGAclear^{™} Kit;
Purification for Large Scale Transcription Reactions.

### 2.3 sgRNA primer design

**Table 6**

| **Mouse strain** | **sgRNA's targeting sequence (5'>3')** | **Designed sgRNA sequence (5'>3')** |
|---|---|---|
| IgK | TCCAGTGAGCAGTTAACAT CTGG (SEQ ID NO:11) | GUCCAGUGAGCAGUUAACAUCguuuaagagcua ugcuggaaacagcauagcaaguuuaaauaaggcuaguccguuau caacuugaaaaaguggcaccgagucggugcuuuuuuu (SEQ ID NO: 3) |

### 2.4 Preparation of sgRNA transcription template

### 2.4.1 Taq-mix PCR system was shown in Table 2 above, with each sample being amplified in 20 µL × 2 tubes.

### 2.4.2 Touchdown program was shown in Table 3 above.

PCR products were recovered with a recovery kit and used for the subsequent experiment, that is, in vitro transcription.

### 2.5 In vitro transcription

### 2.5.1 A transcription system was shown in Table 4 above.

The transcription system was incubated in a 37 °C incubator for 18 h and recovered with a kit known as MEGAclear^{™} Kit Purification for Large Scale Transcription Reactions.

### 2.5.2 Agarose gel electrophoresis result of Igx-sgRNA transcription product was shown in Figure 5. As shown in Figure 5, after the transcription procedure described above, a single transcription product was obtained as Igκ-sgRNA, which can be used for the subsequent introduction.

### 2.6 Introduction of the sgRNA and Cas9 protein to fertilized eggs of host animals:

Mice were subjected to ovulation induction and then in vitro fertilization to obtain fertilized eggs, which were cultured; then, the sgRNA and Cas9 protein were mixed together and electroporated into the fertilized eggs of mice, or Cas9 protein (or Cas9 mRNA, commercially available) along with the sgRNA was injected into the fertilized eggs of mice via microinjection.

The resultant fertilized egg cells were implanted into surrogate female mice, thereby producing F0 generation chimeric mice. By extracting genomic DNA from the mouse tail, performing PCR amplification, and analyzing the PCR amplification products, F0 generation positive knockout mice were identified.

In the above PCR amplification, the used PCR primers KC-nF and KC-nR were designed at both terminals of the deleted sequence. The sequences of the primers were shown in Table 7.

**Table 7**

| **Primer** | **Sequence (5'>3')** |
|---|---|
| KC-nF | CAAGATTGTATATATGTGCATCCTG (SEQ ID NO: 12) |
| KC-nR | CACTGCCATCAATCTTCCACTTGA (SEQ ID NO: 13) |

Analyses of PCR amplification products included agarose gel electrophoresis and sequencing analyses so as to determine whether the target sequence had been deleted. According to the sequencing results of the PCR products, it was determined that the deleted sequence was 76 bp in length. Therefore, the PCR products amplified with the above primers could detect whether the deleted genes or the wild-type genes, depending on the size of the PCR product fragments, wherein for the wild-type genes and those genes with the target sequence deleted, the target bands amplified with the primers KC-nF and KC-nR were 299 bp and 223 bp in size, respectively (the results were not shown).

F0 generation chimeric mice with correct gene knockout were selected for the subsequent reproduction and identification.

### 2.7 Reproduction of heterozygous and homozygous gene knockout mice

F0 generation mice with target gene knockout were mated with wild-type mice to obtain F1 generation mice. By extracting genome from the mouse tail and performing PCR detection, stably heritable gene knockout positive F1 generation heterozygous mice were selected. The F1 generation heterozygous mice were then mated with each other to obtain gene knockout positive F2 generation homozygous mice, i.e., Igκc knockout homozygous mice. The obtained F2 generation mice and their offspring homozygous mice were subjected to genotype identification in the same way as that in step 2.6 above, and the results thereof were shown in Figure 6.

As shown in Figure 6, IgK knockout homozygous mice were indeed obtained following the above procedure.

### Example 3: Design of sgRNAs for deleting a fragment between IgLc2 and IgLc1 of mouse antibody gene light chain Igλ (hereinafter referred to as "IgL gene"), and preparation and identification of gene knockout mice (hereinafter referred to as " IgL mice" or " IgL homozygous mice")

To delete the fragment between IgLc2 and IgLc1 of mouse antibody gene light chain Igλ, one target on the upstream of the exon of mouse IgLc2 gene and one target on the downstream of the exon of mouse IgLc1 gene were selected, respectively, and one sgRNA was designed for each target. Figure 7 shows a schematic diagram of the targets of the sgRNAs, wherein the five-star symbols denote the locations of the upstream and downstream targets, respectively. The sequences of the two designed sgRNAs and their target sequences were shown in Table 8.

### 3. Construction of IgL mice

### 3.1 Specific steps, including: sgRNA primer design, sgRNA in vitro transcription, mouse embryo injection, and positive mouse identification.

### 3.2 Experimental reagents:

MEGAshortscript^{™} Kit;
MEGAclear^{™} Kit;
Purification for Large Scale Transcription Reactions;

### 3.3 sgRNA primer design

**Table 8**

| **Mouse strain** | **sgRNA's targeting sequence (5'>3')** | **Designed sgRNA sequence (5'>3')** |
|---|---|---|
| IgL | GGCCACTGTCACACCACTC GGGG (SEQ ID NO: 14) | GGCCACUGUCACACCACUCGguuuaagagcua ugcuggaaacagcauagcaaguuuaaauaaggcuaguccguu aucaacuugaaaaaguggcaccgagucggugcuuuuuuu (SEQ ID NO: 4) |
| | ACGATCACTGATTTCTACCC AGG (SEQ ID NO: 15) | ACGAUCACUGAUUUCUACCCguuuaagagcua ugcuggaaacagcauagcaaguuuaaauaaggcuaguccguu aucaacuugaaaaaguggcaccgagucggugcuuuuuuu (SEQ ID NO: 5) |

### 3.4 Preparation of sgRNA transcription template

### 3.4.1 Taq-mix PCR system was shown in Table 2 above, with each sample being amplified in 20 µL × 2 tubes.

### 3.4.2 Touchdown program was shown in Table 3 above.

PCR products were recovered with a recovery kit and used for the subsequent experiment, that is, in vitro transcription.

### 3.5 In vitro transcription

### 3.5.1 A transcription system was shown in Table 4 above.

The transcription system was incubated in a 37 °C incubator for 18 h and recovered with a kit known as MEGAclear^{™} Kit Purification for Large Scale Transcription Reactions.

### 3.5.2 Agarose gel electrophoresis result of sgRNA transcription products was shown in Figure 8.

As shown in Figure 8, after the transcription procedure described above, two individual transcription products were obtained, i.e., two sgRNAs designed against the upstream and downstream targets as described above, which can be used for the subsequent introduction.

### 3.6 Introduction of the sgRNA and Cas9 protein to fertilized eggs of host animals:

Mice were subjected to ovulation induction and then in vitro fertilization to obtain fertilized eggs, which were cultured; then, the sgRNAs and Cas9 protein were mixed together and electroporated into the fertilized eggs of mice, or Cas9 protein (or Cas9 mRNA, commercially available) along with the sgRNAs were injected into the fertilized eggs of mice via microinjection.

The resultant fertilized egg cells were implanted into surrogate female mice, thereby producing F0 generation chimeric mice. By extracting genomic DNA from the mouse tail, performing PCR amplification, and analyzing the PCR amplification products, the F0 generation positive knockout mice were identified.

In the above PCR amplification, the used PCR primers LC2-nF1 and IgL-R2 were designed at both terminals of the deleted sequence. The sequences of the primers were shown in Table 9.

**Table 9**

| **Primer** | **Sequence (5>3')** |
|---|---|
| LC2-nF1 | GGTGAGGAAAAGGAAAAAGTGCC (SEQ ID NO: 16) |
| IgL-R | CAAATCCCAAAGTCTGGTTCTGAAG (SEQ ID NO: 17) |
| LC1-nF21 | GCTTGTACCCAGAAGGCATAGATT (SEQ ID NO: 18) |

Analyses of PCR amplification products included agarose gel electrophoresis and sequencing analyses so as to determine whether the target sequence had been deleted. According to the sequencing results of the PCR products, it was determined that the deleted sequence was 137 kp in length. Therefore, the PCR product size of the knockout mice amplified with the above primers was 1170 bp. The PCR primer LC1-nF21 was designed near the 3' end of the deleted sequence, and the primers LC1-nF21 and IgL-R2 were used to detect the target fragment without gene deletion, and the PCR product size was 1424 bp. Depending on the size of the PCR product fragment, it is also possible to determine whether it is a wild-type gene or a gene with the target sequence deleted.

F0 generation chimeric mice with correct gene knockout were selected for the subsequent reproduction and identification.

### 3.7 Reproduction of heterozygous and homozygous gene knockout mice

F0 generation mice with target gene knockout were mated with wild-type mice to obtain F1 generation mice. By extracting genome from the mouse tail and performing PCR detection, stably heritable gene knockout positive F1 generation heterozygous mice were selected. The F1 generation heterozygous mice were then mated with each other to obtain gene knockout positive F2 generation homozygous mice, i.e., IgL knockout homozygous mice. The obtained F2 generation mice and their offspring homozygous mice were subjected to genotype identification in the same way as that in step 3.6 above, and the results thereof were shown in Figure 9.

As shown in Figure 9, IgL knockout homozygous mice were indeed obtained following the above procedure.

### Example 4: Design of sgRNAs for deleting a fragment between IgHM and IgHA-CH1 of mouse antibody gene heavy chain (hereinafter also to as "IgA gene"), and preparation and identification of gene knockout mice (hereinafter referred to as "IgA mice" or "IgA homozygous mice").

To delete the fragment between IgHM and IgHA-CH1 of mouse antibody gene heavy chain, one target on the upstream of the exon of mouse IgHM gene and one target on the downstream of the first exon of mouse IgHA gene were selected, respectively, and one sgRNA was designed for each target. Figure 10 shows a schematic diagram of the targets of the sgRNAs, wherein the five-star symbols denote the locations of the upstream and downstream targets, respectively. The sequences of the two designed sgRNAs and their target sequences were shown in Table 10.

### 4. Construction of IgA mice

4.1 Specific steps, including: sgRNA primer design, sgRNA in vitro transcription, mouse embryo injection, and positive mouse identification.

### 4.2 Experimental reagents:

### 4.2.1 MEGAshortscript^{™} Kit;

### 4.2.2 MEGAclear^{™} Kit;

Purification for Large Scale Transcription Reactions;

### 4.3 sgRNA primer design

**Table 10**

| **Mouse strain** | **sgRNA's targeting sequence (5'>3')** | **Designed sgRNA sequence (5'>3')** |
|---|---|---|
| IgA | GACTCTCTGAGGACCAGA GAGGG (SEQ ID NO: 7) | GACUCUCUGAGGACCAGAGAguuuaagagcu augcuggaaacagcauagcaaguuuaaauaaggcuaguccg uuaucaacuugaaaaaguggcaccgagucggugcuuuuuuu (SEQ ID NO: 1) |
| | GCTCTGGTAAAGAACGTTA GGGG (SEQ ID NO: 19) | GCUCUGGUAAAGAACGUUAGguuuaagagcu augcuggaaacagcauagcaaguuuaaauaaggcuaguccg uuaucaacuugaaaaaguggcaccgagucggugcuuuuuuu (SEQ ID NO: 6) |

### 4.4 Preparation of sgRNA transcription template

4.4.1 Taq-mix PCR system was shown in Table 2 above, with each sample being amplified in 20 µL × 2 tubes.

### 4.4.2 Touchdown program was shown in Table 3 above.

PCR products were recovered with a recovery kit and used for the subsequent experiment, that is, in vitro transcription.

### 4.5 In vitro transcription

4.5.1 A transcription system was shown in Table 4 above.

The transcription system was incubated in a 37 °C incubator for 18 h and recovered with a kit known as MEGAclear^{™} Kit Purification for Large Scale Transcription Reactions.

4.5.2 Agarose gel electrophoresis result of IgA-sgRNA transcription products was shown in Figure 11. As shown in Figure 11, after the transcription procedure described above, two individual transcription products were obtained, i.e., two sgRNAs designed based on the upstream and downstream targets as described above, which can be used for the subsequent introduction.

### 4.6 Introduction of the sgRNAs and Cas9 protein to fertilized eggs of host animals:

Mice were subjected to ovulation induction and then in vitro fertilization to obtain fertilized eggs, which were cultured; then, sgRNAs and Cas9 protein were mixed together and electroporated into the fertilized eggs of mice, or Cas9 protein (or Cas9 mRNA, commercially available) along with the sgRNAs were injected into the fertilized eggs of mice via microinjection.

The resultant fertilized egg cells were implanted into surrogate female mice, thereby producing F0 generation chimeric mice. By extracting genomic DNA from the mouse tail, performing PCR amplification, and analyzing the PCR amplification products by gel electrophoresis and sequencing, F0 generation positive knockout mice were identified.

In the above PCR amplification, the used PCR primers IgA-KO-1F and IgA-KO/WT-1R were designed at both terminals of the deleted sequence. The sequences of the primers were shown in Table 11.

**Table 11**

| **Primer** | **Sequence (5'>3')** |
|---|---|
| IgA-KO-1F | GCAGACTGTGTGCTACAGTGGAGTTTCAA (SEQ ID NO: 20) |
| IgA-KO/WT-1R | CAATTGTGCCAGTTAAGGTGTCAG (SEQ ID NO: 21) |
| IgA-WT-1F | GAAGGATATAACCACCGTAAACTTCCCACCTG (SEQ ID NO: 22) |

The PCR amplification products were analyzed by agarose gel electrophoresis and sequencing, respectively, to determine whether the target sequence had been deleted.

According to the sequencing results of the PCR products, it was determined that the deleted sequence was 163 kb in length. The PCR product size of the gene knockout mice amplified with the above primers was 1170 bp. The PCR primer IgA-KO/WT-1R was designed near the 3' end of the deleted sequence, and the primers IgA-WT-1F and IgA-KO/WT-1R were used to detect the target fragment without gene deletion, and the PCR product size was 724 bp. Depending on the size of the PCR product fragment, it is also possible to determine whether it is a wild-type gene or a gene with the target sequence deleted.

F0 generation chimeric mice with correct gene knockout were selected for the subsequent reproduction and identification.

### 4.7 Reproduction of heterozygous and homozygous gene knockout mice

F0 generation mice with target gene knockout were mated with wild-type mice to obtain F1 generation mice. By extracting genome from the mouse tail and performing PCR detection, stably heritable gene knockout positive F1 generation heterozygous mice were selected. The F1 generation heterozygous mice were then mated with each other to obtain gene knockout positive F2 generation homozygous mice, i.e., IgA knockout homozygous mice. The obtained F2 generation mice and their offspring homozygous mice were subjected to genotype identification in the same way as that in step 4.6 above. Meanwhile, the gene knockout homozygous mouse identified by sequencing were used as a homozygous positive control (the lane labeled "+"). Furthermore, a wild-type mouse control (the lane labeled "-") and a negative control without DNA template (also called a "water control") were also set up, and the results thereof were shown in Figure 12.

As shown in Figure 12, IgA knockout homozygous mice were indeed obtained following the above procedure.

### Example 5: Preparation and identification of IgM, IgK, and IgL triple-gene knockout mice (referred to herein as "IgM-KL mice").

The IgM knockout homozygous mice prepared in Example 1 were mated with the IgK knockout homozygous mice prepared in Example 2 to obtain IgM-IgK knockout heterozygous mice; simultaneously, the IgM knockout homozygous mice prepared in Example 1 were mated with the IgL knockout homozygous mice prepared in Example 3 to obtain IgM-IgL knockout heterozygous mice; then the obtained IgM-IgK knockout heterozygous mice were mated with the IgM-IgL knockout heterozygous mice to obtain IgM(+/+)IgK(+/-)IgL(+/-) mice; finally, the IgM(+/+)IgK(+/-)IgL(+/-) mice were mated with each other to obtain IgM(+/+)IgK(+/+)IgL(+/+) mice, called IgM-KL mice.

The target mouse strain was identified by extracting genomic DNA from the mouse tail, performing PCR amplification, and analyzing the PCR amplification products by gel electrophoresis. The primers for identifying IgM, IgK, and IgL gene deletions were the same as those disclosed in Examples 1, 2, and 3, respectively. Meanwhile, the gene knockout homozygous mouse and heterozygous mouse with the corresponding gene knockout identified by sequencing were used as a homozygous positive control (the lane labeled "+") and a heterozygous control (the lane labeled "+/-"), respectively. Furthermore, a wild-type mouse control (the lane labeled "-") and a negative control without DNA template (also called a "water control") were also set up, and the genotype identification results were shown in Figures 13A, 13B, 13C, respectively.

As shown in Figures 13A, 13B, 13C, IgM, IgK, and IgL triple-gene knockout mice were indeed obtained following the above procedure.

### Example 6: Preparation and identification of IgA, IgK, and IgL triple-gene knockout mice (referred to herein as "IgA-KL mice")

The IgM-KL knockout homozygous mice prepared in Example 5 were mated with the IgA knockout homozygous mice prepared in Example 4 to obtain IgA-KL knockout heterozygous mice; then the obtained IgA-KL knockout heterozygous mice were mated with each other to obtain IgA (+/+) IgK(+/+)IgL(+/+) mice, called IgA-KL mice.

The target mouse strain was identified by extracting genomic DNA from the mouse tail, performing PCR amplification, and analyzing the PCR amplification products by gel electrophoresis. The primers for identifying IgA and IgK deletions were the same as those disclosed in Examples 4 and 2, respectively, and the primers for identifying IgL deletion were shown in Table 12 below. Meanwhile, the gene knockout homozygous mouse with the corresponding gene knockout identified by sequencing was used as a homozygous positive control (the lane labeled "+"). Furthermore, a wild-type mouse control (the lane labeled "-") and a negative control without DNA template (also called a "water control") were also set up, and the genotype identification results were shown in Figures 14A, 14B, 14C, respectively.

**Table 12**

| **Primer** | **Sequence (5>3')** | **PCR product** |
|---|---|---|
| LC2-nF1 | GGTGAGGAAAAGGAAAAAGTGCC (SEQ ID NO: 23) | WT:768bp |
| LC1-seqR | TGGTAGGCAAAAGCACCAAGGAT (SEQ ID NO: 24) | |
| | | mut:514bp |
| LC1-nF21 | GCTTGTACCCAGAAGGCATAGATT (SEQ ID NO: 25) | |

As shown in Figures 14A-C, IgA, IgK, and IgL triple-gene knockout mice were indeed obtained following the above procedure.

### Example 7: Preparation and identification of the genetically modified mice of the present invention, IgM-KL-hIgHD mice

In this example, 6 BACs carrying all human or murine antibody gene sequences to be introduced were introduced into the IgM-KL mice prepared in Example 5, to prepare IgM-KL-hIgHD mice and IgA-KL-hIgHD mice. The six BACs were called CH17-268I9, CTD-3054M17, CTD-2548B8-CZ, RP11-965B13, CH17-185P21-CZ, and RP23-351J19 -CZ, respectively, among which, the four BACs CH17-268I9, CTD-3054M17, CTD- 2548B8-CZ, and RP11-965B13 carried a total of about 70 V genes, CH17-185P21 -CZ contained the complete human D region genes and J region genes, and RP23-351J19-CZ contained murine IgHG2c (a complete IgHG2c region for producing conventional antibodies; a IgHG2c fragment without CH1 for producing nanobodies; in this example, the IgHG2c fragment without CH1 was introduced), IgHE, IgHA, and LCR region (35 kb) (for detailed information of the genes carried by each BAC, please see Table 13); the two parts above were connected to each other via a Switch region of the murine IgHM (i.e., between human J region and murine IgHG2c genes).

Of the six BAC clones above, CH17-268I9, CTD-3054M17, and RP11-965B13 were the original BAC clones purchased from INVITROGEN (Shanghai) Trading Co. Ltd., and while CTD-2548B8-CZ, RCH17-185P21-CZ, and RP23-351J19-CZ were obtained by making some modifications on the basis of their respective original BAC clone strains (i.e., CTD-2548B8, RCH17-185P21, and RP23-351J19, also purchased from INVITROGEN (Shanghai) Trading Co. Ltd.), and the specific modification method was as follows.

Firstly, the blank BAC strain was prepared into electrocompetent cells. Then, pKD46-Tet plasmids (Wuhan Miaoling Bio Co., Ltd., P7957) were electroporated into the electrocompetent cells. The fragments to be recombined (as shown in SEQ ID NO:76-78, respectively) were constructed by OverLap-PCR or enzymatic ligation. The BAC bacteria carrying pKD46-Tet plasmids were prepared into electrocompetent cells, and then the fragments to be recombined were electrotransferred into the competent cells. Subsequently, screening was carried out by using the corresponding antibiotics, and the positive clones were verified by colony PCR. Finally, the positive BAC clones were extracted from the bacteria and determined by Fast-NGS sequencing.

**Table 13. Detailed information of genes carried by each BAC**

| BAC Clones No. | CH17-268I9 | CTD-3054M17 | CTD-2548B8-CZ | RP11-965B13 | CH17-185P21-CZ | RP23-351J19 -CZ |
|---|---|---|---|---|---|---|
| Important sequence contained | hIGHV3-60 | hIGHV3-41 | hIGHV3-23 | hIGHV3-11 | HIGHV1-2 | mIgHG2c-CH1 |
| | hIGHV4-59 | hIGHV4-39 | hIGHV3-22 | hIGHV5-10-1 | hIGHV6-1 | mIgHE |
| | hIGHV1-58 | hIGHV3-38 | hIGHV3-21 | hIGHV3-64D | hIGHD1-1 | mIgHA |
| | IGHV3-57 | hIGHV3-37 | hIGHV3-20 | hIGHV3-7 | hIGHD2-2 | mLCR |
| | hIGHV7-56 | hIGHV3-36 | hIGHV3-19 | hIGHV3-6 | hIGHD3-3 | |
| | hIGHV4-55 | hIGHV3-35 | hIGHV1-18 | hIGHV2-5 | hIGHD4-4 | |
| | hIGHV3-54 | hIGHV7-34-1 | hIGHV1-17 | hIGHV7-4-1 | hIGHD5-5 | |
| | hIGHV3-53 | hIGHV4-34 | hIGHV3-16 | hIGHV4-4 | hIGHD6-6 | |
| | IGHV3-52 | hIGHV3-33-2 | hIGHV3-15 | hIGHV1-3 | HIGHD1-7 | |
| | hIGHV5-51 | hIGHV3-33 | hIGHV1-14 | hIGHV1-2 | hIGHD2-8 | |
| | hIGHV3-50 | hIGHV3-32 | hIGHV3-13 | hIGHV6-1 | hIGHD3-9 | |
| | hIGHV3-49 | MGHV4-30-2 | hIGHV1-12 | | hIGHD3-10 | |
| | hIGHV3-48 | hIGHV3-30-2 | hIGHV3-11 | | hIGHD4-11 | |
| | hIGHV3-47 | IGHV3-30 | | | hIGHD5-12 | |
| | hIGHV1-46 | hIGHV3-29 | | | hIGHD6-13 | |
| | hIGHV1-45 | hIGHV4-28 | | | hIGHD1-14 | |
| | hIGHV3-43 | hIGHV7-27 | | | hIGHD2-15 | |
| | hIGHV3-42 | hIGHV2-26 | | | hIGHD3-16 | |
| | hIGHV3-41 | hIGHV3-25 | | | hIGHD4-17 | |
| | hIGHV4-39 | hIGHV1-24 | | | hIGHD5-18 | |
| | hIGHV3-38 | hIGHV3-23 | | | hIGHD6-19 | |
| | hIGHV3-37 | hIGHV3-22 | | | hIGHD1-20 | |
| | | | | | hIGHD2-21 | |
| | | | | | hIGHD3-22 | |
| | | | | | hIGHD4-23 | |
| | | | | | hIGHD5-24 | |
| | | | | | hIGHD6-25 | |
| | | | | | hIGHD1-26 | |
| | | | | | hIGHD7-27 | |
| | | | | | hIGHJ1P | |
| | | | | | hIGHJ1 | |
| | | | | | hIGHJ2 | |
| | | | | | hIGHJ2P | |
| | | | | | hIGHJ3 | |
| | | | | | hIGHJ4 | |
| | | | | | hIGHJ5 | |
| | | | | | hIGHJ3P | |
| | | | | | hIGHJ6 | |

In Table 13, "mIgHG2c-CH1" represents mIgHG2c gene sequence without the CH1 fragment.

The specific procedure was as follows:
(1) 6 BACs carrying all the human antibody gene sequences to be introduced were introduced into the IgM-KL mice prepared in Example 5; specifically, BAC plasmids were extracted firstly, and then the BAC framework was cut off with the corresponding restriction endonuclease followed by purification; the purified BAC genes were then mixed in equal molar amounts to prepare an injection solution, which was then injected into the masculonucleus of fertilized eggs through the pronucleus, and finally the fertilized eggs were transplanted into the surrogate mice to obtain F0 generation mice with human antibody genes introduced. Subsequently, the introduced antibody gene sequences were identified by PCR. The primers used were shown in Table 14.

**Table 14**

| **Primer number** | **Primer name** | **Sequence (5'>3')** | **The size of PCR products** |
|---|---|---|---|
| 1 | H1-F | TCACCAGGGAGTTTGTGCTAAGCTG (SEQ ID NO: 26) | 967bp |
| | H1-R | GGAGAAGGGTCAGACGTGAAACCTG (SEQ ID NO: 27) | |
| 2 | H2-F | ACAACTCAAGTGTCCATCGACTGCC (SEQ ID NO: 28) | 1155bp |
| | H2-R | CTCAGCACCCACTGCTGTTCTGTAA (SEQ ID NO: 29) | |
| 3 | H3-F | TTCCCATGTGAGCCAGTTTCCTTCC (SEQ ID NO: 30) | 953bp |
| | H3-R | GGCTGCACCGTTATTTCCTCTGTCT (SEQ ID NO: 31) | |
| 4 | H4-F | AGAGGGCAAGGCTCAGGAAAAGTTC (SEQ ID NO: 32) | 1344bp |
| | H4-R | ACCGATTTATGACACTGGTGCAGGG (SEQ ID NO: 33) | |
| 5 | H5-F | GGACACTGTACAACCCATCTGTGCTT (SEQ ID NO: 34) | 850bp |
| | H5-R | GACGTGGTCTATTCTGTTGTCCCACC (SEQ ID NO: 35) | |
| 6 | H6-F | TACATTGTGTGAGTGACAGGGCAGTG (SEQ ID NO: 36) | 879bp |
| | H6-R | ATGAGGAGATGTGCTCTCAGGGGATT (SEQ ID NO: 37) | |
| 7 | H7-F | CCAAGGACACTCTCATCTGCCC (SEQ ID NO: 38) | 990bp |
| | H7-R | CCATGGTTGAGGAGTTTTCATCTC (SEQ ID NO: 39) | |
| 8 | H8-F | GGGAATGCAAATCTCCACCAGCT (SEQ ID NO: 40) | 763bp |
| | H8-R | CAAACAGGACTCAGCTTCTGCTTC (SEQ ID NO: 41) | |
| 9 | H9-F | GCAGTCACCAGAGCTCCAGACAAT (SEQ ID NO: 42) | 625bp |
| | H9-R | CAGTTTAAGACACAGCAAGAAGTTCCA (SEQ ID NO: 43) | |
| 10 | H10-F | CACTGAAAGAAATCGGCCTCCAGTG (SEQ ID NO: 44) | 889bp |
| | H10-R | GTGGGGTGATGTCTTGGGGAGAGTC (SEQ ID NO: 45) | |
| 11 | H11-F | TTCATGCCCCTAGAGTTGGCTGAAG (SEQ ID NO: 46) | 721bp |
| | H11-R | TGGAGTGGAGCTCTGGTCTAGTGAC (SEQ ID NO: 47) | |

(2) the F0 generation mice with human antibody gene sequences introduced obtained in step (1) were mated with IgM-KL homozygous mice to obtain F1 generation heterozygous mice. By extracting genomic DNA from the mouse tail and performing PCR detection, stably heritable gene recombined positive F1 generation heterozygous mice were selected. The F1 generation heterozygous mice were then mated with each other to obtain gene recombined positive F2 generation homozygous mice, i.e., IgM-KL-hIgHD homozygous mice. The obtained F2 generation homozygous mice were subjected to genotype identification in the same way as that in step 1 above. At the same time, the IgM-KL background mice were detected by PCR, and the identification method was the same as that in Example 5.

The results were shown in Fig. 15.

The above results showed that all the above genes were successfully introduced, i.e., IgM-KL-hIgHD mice were successfully obtained in this example.

### Example 8: Preparation and identification of the genetically modified mice of the present invention, IgA-KL-hIgHD mice

In this example, the IgM-KL-hIgHD mice prepared in Example 7 were mated with the IgA-KL homozygous mice to obtain F1 generation mice, i.e., IgA-KL-hIgHD heterozygous mice; then the F1 generation heterozygous mice were mated with each other to obtain F2 generation homozygous mice, i.e., IgA-KL-hIgHD mice.

The results of genotype identification of the F2 generation IgA-KL-hIgHD mice were shown in Figure 16.

The above results showed that all the above genes were successfully introduced, i.e., IgA-KL-hIgHD mice were successfully obtained in this example.

### Example 9: Phenotype detection of IgM gene edited mice

(I) Antigen immune response and titer detection of IgM knockout homozygous mice Three IgM knockout homozygous mice (prepared in Example 1) were individually immunized with OVA (chicken ovalbumin, purchased from Beijing Borsee Science and Technology Co., Ltd.) as an antigen, and the procedure was specifically as follows:
   Mice aged 6-8 weeks were selected. The antigen was mixed with an equal volume of Freund's complete adjuvant and emulsified until it no longer dissolved in water. Then the emulsion can be injected subcutaneously at multiple sites into the mice. For the primary immunization, the injection dose was 50 µg per mouse and the injection volume was 0.2 mL per mouse.

Two weeks after the primary immunization, a second immunization was carried out subcutaneously. The antigen was emulsified with an equal volume of Freund's incomplete adjuvant and then injected subcutaneously at multiple sites into the mice. The injection dose was reduced to 25 µg per mouse and the injection volume was 0.2 mL per mouse.

Blood samples were collected on days 0, 17, and 24, respectively, a plate was coated with goat anti-mouse IgM polyclonal antibodies, the antigen was labeled with biotin, and the IgM antibody titer in serum was detected using HRP-Streptavidin. The antigen immune titer assay results of the three IgM knockout homozygous mice were shown in Figures 17A, 17B, and 17C, respectively.

As shown in Figures 17A-C, the IgM antibody titer of serum from the three IgM knockout homozygous mice subjected to three immunizations by OVA had increased somewhat, but the overall titer was very low.

### (II) Western Blot assay of serum from IgM gene knockout mice immunized with CRP

CRP (i.e., human C-reactive protein, purchased from Beijing Biobridge Biotechnology Co.,Ltd.) antigen affinity column material was prepared according to the instructions of CNBr-activated SepharoseTM 4B (purchased from GE, No. 17043001), and the volume of CRP-Sepharose packing prepared using 1.5 mg of CRP antigen was set to 1.5 mL.

One C57BL/6 wild-type mouse (purchased from Viton Lever), one IgM knockout heterozygous mouse (the F1 generation heterozygous mouse prepared in Example 1), and one IgM knockout homozygous mouse (the F2 generation homozygous mouse prepared in Example 1), each aged 6-8 weeks, were immunized with CRP, and the immunization process was the same as that with OVA immunization. On the day 7 after the third immunization, 20-50 µL of mouse blood was collected, and placed at room temperature for about 30 min until the blood was coagulated, and the serum was collected after centrifugation. 1 µL of each sample was taken separately, to which 100 µL of PBS was added, followed by addition of 10 µL of CRP-Sepharose packing. After reacting at room temperature for 60 min, centrifugation was carried out at 6000 rpm for 30 seconds, and then the supernatant was discarded. The packing was washed with PBS for 3 times, resuspended with 10 µL PBS and boiled, subjected to 12% SDS-PAGE electrophoresis, and transferred onto a PVDF membrane and blocked, then reacted with goat anti-mouse IgM antibody (Sigma, ISO2-1KT) and further color developed, and the results were shown in Figure 18.

As shown in Figure 18, after immunization with CRP antigen, IgM antibodies that react specifically with the antigen CRP were detected in sera of C57BL/6 wild-type mice (lane 1), IgM CH1 ko heterozygous mice (lane 2), and IgM CH1 ko homozygous mice (lane 3), and the size of homozygous mouse IgM heavy chain was 60 KD under reducing conditions, significantly smaller than that of wild-type mouse IgM heavy chain (78KD), indicating that the CH1 domain of IgM homozygous mouse strain has been successfully knocked out.

### (III) RT-PCR detection of IgM gene knockout mice immunized with CRP

Splenocytes were taken from the IgM knockout homozygous mice after the third immunization with CRP antigen in the step (II) above, and total RNA was extracted with Trizol and subjected to reverse transcription to cDNA. With the cDNA as a template, the variable region and part of the CH2 gene linked thereto were amplified using primers specific for IgM subtype antibody, with the upstream primer being MHV1-12 mixed primers and the downstream primer being IgHM-CH2-R4. The primers used and their sequences were shown in Table 15, wherein the variable bases S, Y, R, W, M, and K were as defined in the prior art; specifically, S was G or C, Y was C or T, R was A or G, W was A or T, M was A or C, and K was G or T.

**Table 15**

| **Primer** | **Sequence (5'>3')** |
|---|---|
| MHV1 | ATGAAATGCAGCTGGGGCATSTTCTTC (SEQ ID NO: 48) |
| MHV2 | ATGGGATGGAGCTRTATCATSYTCTT (SEQ ID NO: 49) |
| MHV3 | ATGAAGWTGTGGTTAAACTGGGTTTTT (SEQ ID NO: 50) |
| MHV4 | ATGRACTTTGGGYTCAGCTTGRTTT (SEQ ID NO: 51) |
| MHV5 | ATGGGACTCCAGGCTTCAATTTAGTTTTCCTT (SEQ ID NO: 52) |
| MHV6 | ATGGCTTGTCYTTRGSGCTRCTCTTCTGC (SEQ ID NO: 53) |
| MHV7 | ATGGRATGGAGCKGGRGTCTTTMTCTT (SEQ ID NO: 54) |
| MHV8 | ATGAGAGTGCTGATTCTTTTGTG (SEQ ID NO: 55) |
| MHV9 | ATGGMTTGGGTGTGGAMCTTGCTTATTCCTG (SEQ ID NO: 56) |
| MHV10 | ATGGGCAGACTTACCATTCTCATTCCTG (SEQ ID NO: 57) |
| MHV11 | ATGGATTTTGGGCTGATTTTTTTTATTG (SEQ ID NO: 58) |
| MHV12 | ATGATGGTGTTAAGTCCTTCTGTACC (SEQ ID NO: 59) |
| IgHM-CH2-R4 | GTTCATCTCTGCGACAGC (SEQ ID NO: 60) |

The PCR reaction system was shown in Table 16.

**Table 16**

| | |
|---|---|
| Template cDNA | 5 µL |
| MHV1-12 equivalent mixture | 2 µL |
| Downstream primers (ie, IgHM-CH2-R4) | 2 µL |
| I-5^{™} 2 × High-Fidelity Master Mi (Tsingke, #:TP001) | 25 µL |
| ddH₂O | 16 µL |

The PCR reaction procedure was shown in Table 17.

**Table 17**

| | | |
|---|---|---|
| 98°C | 2 min | 1 cycle |
| 98°C | 10 s | 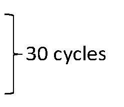 |
| 50°C | 20 s | |
| 72°C | 30s | |
| 72°C | 5 min | 1 cycle |

The PCR amplification products were ligated into a pEASY^{®}-Blunt Zero Cloning vector, and then transformed into TOP10 strain and plated (ampicillin resistant). One clone was picked for colony PCR, and the results were shown in Figure 19A. This positive clone was sequenced. As can be seen from the sequencing results, the IgM antibody heavy chain variable region FR4 expressed in the IgM homozygous mice was directly linked to CH2 (the starting amino acid sequence AVAEMN), and partial sequencing results and the analysis thereof were shown in Figure 19B, in which the sequence was indicated in SEQ ID NO:79. This indicates that the CH1 exon of genomic IgM has been successfully knocked out.

### Example 10: Phenotype detection of IgA gene edited mice

(I) Antigen immune response and titer detection of IgA knockout homozygous mice
   IgA knockout homozygous mice (prepared from Example 4) were immunized with CRP (human C-reactive protein) and OVA (chicken ovalbumin) as an antigen. The immune method was as follows:

Mice aged 6-8 weeks were selected. The antigen was mixed with an equal volume of Freund's complete adjuvant and emulsified until it no longer dissolved in water. Then the emulsion can be injected subcutaneously at multiple sites into the mice. For the primary immunization, the injection dose was 50 µg per mouse and the injection volume was 0.2 mL per mouse. After the primary immunization, subsequent subcutaneous immunizations were carried out every two weeks. The antigen protein was emulsified with an equal volume of Freund's incomplete adjuvant and then injected subcutaneously at multiple sites into the mice. The injection dose was reduced to 25 µg per mouse and the injection volume was 0.2 mL per mouse.

The serum titer detection method was performed as follows: the antigen protein was diluted to 2 µg/mL, 100 µL of the dilution was taken and added to a polystyrene enzyme-linked plate for plating, and the IgA antibody that specifically binds to the antigen protein in serum was detected using Biotin-goat anti-mouse IgA (Abeam, ab97231).

The results were shown in Figure 20A and Figure 20B. Specifically, blood samples were collected on the 8th day after the third immunization with the antigen protein, and then subjected to ELISA assay for serum titers. The results showed that almost no IgA antibodies specifically binding to the antigen protein were detected in IgA-CH1-KO homozygous mice, and the serum titers in all cases did not exceed 1:400.

(II) Western Blot assay of serum from IgA knockout homozygous mice immunized with CRP CRP antigen affinity column material was prepared according to the instructions of CNBr-activated SepharoseTM 4B (purchased from GE, No. 17043001), and the volume of CRP-Sepharose packing prepared using 1.5 mg of CRP antigens was set to 1.5 mL.

Two C57BL/6 wild-type mice and one IgA knockout homozygous mouse (prepared from Example 4), each aged 6-8 weeks, were immunized with CRP, i.e. human C-reactive protein, and the immunization procedure was the same as that in the above step (I). On the day 7 after the third immunization, 20-50 µL of mouse blood was collected, and placed at room temperature for about 30 min until the blood was coagulated, and the serum was collected after centrifugation. 2 µL of each sample was taken separately, to which 100 µL of PBS was added, followed by addition of 10 µL of CRP-Sepharose packing. After reacting at room temperature for 60 min, centrifugation was carried out at 6000 rpm for 30 seconds, and then the supernatant was discarded. The packing was washed with PBS for 3 times, resuspended with 10 µL PBS and boiled, subjected to 12% SDS-PAGE electrophoresis, and transferred onto a PVDF membrane and blocked, then reacted with goat anti mouse IgG Fc HRP (JACKSON,115-035-071) and further color developed. According to the design of the present application, since the mouse genes IgM, IgD, IgG, and IgE were knocked out and the CH1 gene of IgA heavy chain was knocked out, immunization of IgA mice with an antigen protein will only produce IgA heavy chain antibodies (without the CH1 domain), and the molecular weight of one single heavy chain of IgA was about 40 KD. Antibodies produced by C57BL/6 wild-type mice and IgA homozygous mice after immunization with the antigen protein were separated, electrophoresed, and color developed, and the results were shown in Figure 21. Figure 21 showed Western Blot results of mouse sera under reducing conditions, wherein lane 1 and lane 2 represented the immunized C57BL/6 mouse serum samples, and lane 3 represented the immunized IgA homozygous mouse serum sample. The results showed that the antibodies produced by IgA homozygous mice were consistent with the expectation, indicating that the gene fragment between CH1 and IgHA-CH1 of mouse genomic IgHM has been successfully knocked out.

### (III) RT-PCR detection of splenocytes from IgA homozygous mice immunized with CRP

Splenocytes were taken from the IgA homozygous mice after the third immunization with CRP antigen as described in the step (II) above, total RNA was extracted with Trizol and subjected to reverse transcription to cDNA. With the cDNA as a template, the variable region and part of the CH2 gene linked thereto were amplified using primers specific for IgA subtype antibody, with the upstream primer being MHV1-12 mixed primers (the sequences thereof were shown in table 15 above) and the downstream primer being IgHA-CH2-R3 (the sequence was: CTGCATCCTTCCCAGTGGAG, i.e., SEQ ID NO: 61)

The PCR reaction system was the same as that shown in Table 16 above (except for the different downstream primer).

The PCR reaction procedure was the same as that shown in Table 17 above.

The PCR amplification products were ligated into a pEASY^{®}-Blunt Zero Cloning vector, and then transformed into TOP10 strain and plated (ampicillin resistant). Four clones were picked for colony PCR, and the results were shown in Figure 22A. The four positive clones were sequenced, and partial sequencing results and the analyses thereof were shown in Figure 22B, in which the sequences of #1, #2, #3, and #4 were illustrated in SEQ ID NOs: 80, 81, 82, and 83, respectively. As can be seen from the partial sequencing results, IgA antibody heavy chain variable region FR4 expressed in the IgA homozygous mice was directly linked to CH2 (the starting amino acid sequence: GPPPPCPPCPP, SEQ ID NO: 75), indicating that the gene fragment between CH1 of IgHM and IgHA-CH1 of mouse genome has been successfully knocked out.

### Example 11: Phenotype detection of IgM-KL&IgA-KL homozygous mice

### (I) Antigen immune response and titer detection of IgM-KL&IgA-KL homozygous mice

Wild-type C57BL/6, IgM-KL homozygous mice (prepared from Example 5), IgA homozygous mice (prepared from Example 4, as a control), and IgA-KL homozygous knockout mice (prepared from Example 6) were immunized with OVA (chicken ovalbumin), respectively, with the specific procedure as follows:
Mice aged 6-8 weeks were selected. The antigen was mixed with an equal volume of Freund's complete adjuvant and emulsified until it no longer dissolved in water. Then the emulsion can be injected subcutaneously at multiple sites into the mice. For the primary immunization, the injection dose was 50 µg per mouse and the injection volume was 0.2 mL per mouse.

Two weeks after the initial immunization, a second immunization was carried out subcutaneously. The antigen was emulsified with an equal volume of Freund's incomplete adjuvant and then injected subcutaneously at multiple sites into the mice. The injection dose was reduced to 25 µg per mouse and the injection volume was 0.2 mL per mouse.

The serum titer detection was performed as follows: the antigen protein was diluted to 2 µg/mL, and 100 µL of the dilution was taken and added to a polystyrene enzyme-linked plate for plating. For the IgM-KL mice, goat anti-mouse IgM antibody (Sigma, ISO2-1KT) was used, and for the IgA-KL mice, Biotin-goat anti-mouse IgA (Abeam, ab97231) was used, to detect the IgA antibody that specifically binds to the antigen protein in serum.

The results were shown in Figure 23A and Figure 23B. Specifically, blood samples were collected on the 8th day after the third immunization with the antigen protein, and then subjected to ELISA assay for the serum titer. The results showed that antibodies that specifically bind to the antigen protein basically did not occur in both IgM-KL and IgA-KL homozygous mice, in which none of the serum titer exceeded 1:400.

### (II) Western Blot assay of serum from IgM-KL & IgA-KL homozygous mice immunized with OVA

Sera from four kinds of mice, IgM, IgM-KL, IgA, and IgA-KL, after three immunizations using OVA were taken and subjected to Coomassie Brilliant Blue staining to examine their protein abundance. The results were shown in Fig. 24A.

The loading volume of the serum samples in each group of tests was 0.5 µL. After boiling for denaturation, the samples were loaded onto a 12% SDS-PAGE gel for electrophoresis, and transferred onto a PVDF membrane and blocked, the goat anti-mouse Kappa polyclonal antibody (HRP*Polyclonal Goat Anti-Mouse Kappa, C030214) and the goat anti-mouse Lamda polyclonal antibody (HRPPolyclonal Goat Anti-Mouse Lamda, C030213) from Leibochem (Shanghai) Biochemical Technology Co., Ltd. were used to detect the expression of light chain proteins in the mouse sera as described above. The results were shown in Figures 24B and 24C.

As can be seen in Figures 24B and 24C, after antigen immunization, Kappa and Lamda antibodies reacting specifically with the antigen OVA were detected in IgM (lane 1) and IgA mice (lane 3), and no Kappa and Lamda antibodies reacting specifically with the antigen OVA were detected in both IgM-KL (lane 2) and IgA-KL mice (lane 4), indicating that the light chain proteins in IgM-KL and IgA-KL mice had been completely knocked out.

### Example 12: Phenotype detection of IgM-KL- -hIgHD homozygous mice

### (I) Antigen immune response and titer detection of IgM-KL-hIgHD homozygous mice

IgM-KL-hIgHD homozygous mice were immunized with GCC-mFc protein. Specifically, mice aged 6-8 weeks were selected. The antigen was mixed with an equal volume of Freund's complete adjuvant and emulsified until it no longer dissolved in water. Then the emulsion can be injected subcutaneously at multiple sites into the mice. For the primary immunization, the injection dose was 100 µg per mouse and the injection volume was 200 µL per mouse.

Two weeks after the primary immunization, a second immunization was carried out subcutaneously. The antigen was emulsified with an equal volume of Freund's incomplete adjuvant and then injected subcutaneously at multiple sites into the mice. The injection dose was reduced to 50 µg per mouse and the injection volume was 200 µL per mouse. The specific immunization scheme was shown in Table 18.

**Table 18**

| IgM-KL-hIgHD ×3♂ | immunization | Time | Notes |
|---|---|---|---|
| 100 µg GCC-mFc/200µL/animal (Freund's complete adjuvant) | primary immunization | 2023-01-19 | immuned subcutaneously at multiple points |
| 50 µg GCC-mFc/200µL/animal (Freund's incomplete adjuvant) | Second immunization | 2/3/2023 | immuned subcutaneously at multiple points |
| 50 µg GCC-mFc/200µL/animal (Freund's incomplete adjuvant) | Third immunization | 2/17/2023 | immuned subcutaneously at multiple points |
| | | 2/24/2023 | The serum was taken and subjected to titer detection by ELISA. |
| 50 µg GCC-mFc/200µL/animal (Freund's incomplete adjuvant) | Forth immunization | 2/24/2023 | immuned subcutaneously at multiple points |
| 50 µg GCC-mFc/200µL/animal (Freund's incomplete adjuvant) | Fifth immunization | 2023-03-03 | immuned subcutaneously at multiple points |
| | | 3/10/2023 | The serum was taken and subjected to titer detection by ELISA. |

The serum titer detection was performed as follows: the antigen protein was diluted to 2 µg/mL, 100 µL of the dilution was taken and added to a polystyrene enzyme-linked plate for plating, and the humanized nanobody that specifically binds to the antigen protein in the serum from IgM-KL-hIgHD mice was detected using goat anti mouse IgG Fc HRP (JACKSON, 115-035-071) antibody.

The results were shown in Figure 25. Blood was collected on the 7th day after the fifth immunization with the antigen protein, and ELISA detection of serum titers showed that antibodies that specifically bind to the antigen protein occurred in the IgM-KL-hIgHD homozygous mice, among which the serum titers all exceeded 1:24000.

### (II) Western Blot assay of serum from the IgM-KL-hIgHD homozygous mice immunized with GCC-mFc

Sera from IgM-KL-hIgHD mice (#3492, #3493, #3304) after the fifth immunization using GCC-mFc and unimmunized C57BL/6 mice (#3347) were taken for Western assay to verify whether human-mouse chimeric single heavy chain antibody was expressed in the IgM-KL-hIgHD homozygous mice.

The loading volume of the serum samples in each group of tests was 0.5 µL. After boiling for denaturation, the samples were loaded onto a 12% SDS-PAGE gel for electrophoresis, and transferred onto a PVDF membrane and blocked, and goat anti-mouse IgG Fc HRP (JACKSON, 115-035-071) was used to detect the expression of human-mouse chimeric nanobodies in the mouse serum as described above. The results were shown in Figure 26.

As shown in Figure 26, human-mouse chimeric nanobodies that react specifically with the antigen GCC-mFc were detected in the IgM-KL-hIgHD (#3492, #3493, and #3304) immunized with GCC-mFc antigen, and the intact mouse antibody proteins were detected in the unimmunized C57BL/6 mice (#3347), demonstrating that human-mouse chimeric single heavy chain antibodies were expressed in IgM-KL-hIgHD mice.

(III) Human variable region gene V(D)J rearrangement in IgM-KL-hIgHD homozygous mice 1) NGS sequencing analysis of sequences of mouse heavy chain variable region genes One unimmunized mouse and one GCC-mFc-immunized IgM-KL-hIgHD homozygous mouse were selected, and splenocytes from the mice were taken for RNA extraction, and then RNA was reverse transcribed into cDNA using a reverse transcription kit (Takara, 6110A). PCR amplification was performed by using the combination of 10 primers for human V genes with three specific primers for human J genes, respectively, with the cDNA of the sample as a template, to obtain the heavy chain variable region fragments which were then subjected to subsequent sequencing. The sequences of the human gene specific primers were shown in Table 19.

**Table 19**

| Human VH up primers: for nanobody library | Sequence (5'>3') |
|---|---|
| hVH-F1 | GAGGTGCAGCTGGTGCAG (SEQ ID NO: 62) |
| hVH-F2 | GAGGTGCAGCTGGTGGAG (SEQ ID NO: 63) |
| hVH-F3 | GAGGTGCAGCTGTTGGAG (SEQ ID NO: 64) |
| hVH-F4 | CAGGTGCAGCTGCAGGAG (SEQ ID NO: 65) |
| hVH-F5 | CAGGTGCAGCTACAGCAGTG (SEQ ID NO: 66) |
| hVH-F6 | CAGGTTCAGCTGGTGCAG (SEQ ID NO: 67) |
| hVH-F7 | CAGGTCCAGCTGGTACAG (SEQ ID NO: 68) |
| hVH-F8 | GAAGTGCAGCTGGTGGAG (SEQ ID NO: 69) |
| hVH-F9 | CAGGTACAGCTGCAGCAG (SEQ ID NO: 70) |
| hVH-F10 | CAGCTGCAGCTGCAGGAG (SEQ ID NO: 71) |

| Human VH down primers: for nanobody library | Sequence (5'>3') |
|---|---|
| hVH-R1 | TGAGGAGACGGTGACCAG (SEQ ID NO: 72) |
| hVH-R2 | TGAAGAGACGGTGACCATTG (SEQ ID NO: 73) |
| hVH-R3 | TGAGGAGACGGTGACCGTG (SEQ ID NO: 74) |

Since the PCR products of the two samples required NGS sequencing and data analysis, specific barcode sequences needed to be added at the 5' end of primers in Table 16. Therefore, AGTGCT was added to the 5' end of the primer F, and AGACTG was added to the 5 'end of the primer R used for the IgM-KL-hIgHD unimmunized mice; GTCTAA was added to the 5 'end of the primer F and ATTACT was added to the 5' end of the primer R used for the IgM KL-hIgHD immunized mice. The samples of IgM-KL-hIgHD were amplified by PCR using primers added with barcode sequences, and the results were shown in Figure 27. In Figure 27, the PCR products on row 1 and row 2 were PCR products from IgM-KL-hIgHD unimmunized samples; the PCR products on row 3 and row 4 were those from IgM-KL-hIgHD immunized mouse samples.

Next, the PCR products were subjected to NGS sequencing.

### 2) Analysis of NGS sequencing results

The sequencing results and human immunoglobulin sequences were analyzed by bioinformatics technology to identify the expression of human VH, DH, JH genes after V(D)J recombination. As a result, of the 1323424 valid sequencing Reads from the unimmunized IgM-KL-hIgHD mouse samples, the expression of most VH gene fragments, all DH genes, and all JH gene fragments were detected (Table 20). Among these gene fragments, some VH genes were located near the constant region, while others were farther from it. The data of Table 20 indicated that human VH, DH, and JH genes on the human-mouse chimeric nanobody genes transferred by the hIgHD scheme can be rearranged and expressed in IgM-KL background mice.

**Table 20**

| IgHV gene | Count | Ratio | | IgHD gene | Count | Ratio | | IgHJ gene | Count | Ratio |
|---|---|---|---|---|---|---|---|---|---|---|
| V5-51 | 380113 | 28.72% | | IGHD7-27*01 | 312955 | 28.82% | | IGHJ3*02 | 772783 | 58.39% |
| V4-34 | 218868 | 16.54% | | IGHD1-26*01 | 106487 | 9.81% | | IGHJ4*01 | 346550 | 26.19% |
| V4-39 | 213324 | 16.12% | | IGHD6-19*01 | 86790 | 7.99% | | IGHJ2*01 | 75947 | 5.74% |
| V1-24 | 151128 | 11.42% | | IGHD6-13*01 | 80588 | 7.42% | | IGHJ5*02 | 44259 | 3.34% |
| V1-2 | 84436 | 6.38% | | IGHD3-16*01 | 63966 | 5.89% | | IGHJ6*01 | 39624 | 2.99% |
| V3-48 | 50394 | 3.81% | | IGHD3-10*01 | 61724 | 5.68% | | IGHJ5*01 | 20953 | 1.58% |
| V3-23 | 47859 | 3.62% | | IGHD2-15*01 | 55466 | 5.11% | | IGHJ1*01 | 12944 | 0.98% |
| V6-1 | 47322 | 3.58% | | IGHD1-1*01 | 51666 | 4.76% | | IGHJ3*01 | 10316 | 0.78% |
| V4-59 | 42412 | 3.20% | | IGHD3/OR15-3a*01 | 32412 | 2.98% | | IGHJ6*04 | 48 | 0.00% |
| V3-33 | 14310 | 1.08% | | IGHD5-18*01 | 32354 | 2.98% | | | | |
| V4-4 | 13874 | 1.05% | | IGHD1-7*01 | 30929 | 2.85% | | | | |
| V3-21 | 11925 | 0.90% | | IGHD3-22*01 | 22176 | 2.04% | | | | |
| V3-30 | 10300 | 0.78% | | IGHD1-20*01 | 18000 | 1.66% | | | | |
| V3-43 | 8035 | 0.61% | | IGHD6-6*01 | 16512 | 1.52% | | | | |
| V5-10-1 | 5619 | 0.42% | | IGHD3-3*01 | 13732 | 1.26% | | | | |
| V3-11 | 4893 | 0.37% | | IGHD1-14*01 | 10365 | 0.95% | | | | |
| V3-53 | 4517 | 0.34% | | IGHD5-12*01 | 9682 | 0.89% | | | | |
| V3-7 | 3578 | 0.27% | | IGHD3-10*03 | 9416 | 0.87% | | | | |
| V4-30-2 | 3446 | 0.26% | | IGHD4/OR15-4a*01 | 7898 | 0.73% | | | | |
| V3-49 | 2530 | 0.19% | | IGHD4-17*01 | 7557 | 0.70% | | | | |
| V3-15 | 2047 | 0.15% | | IGHD2-21*02 | 7543 | 0.69% | | | | |
| V1-18 | 962 | 0.07% | | IGHD3-9*01 | 6767 | 0.62% | | | | |
| V3-13 | 494 | 0.04% | | IGHD2-2*01 | 6371 | 0.59% | | | | |
| Vl-46 | 429 | 0.03% | | IGHD2-21*01 | 4809 | 0.44% | | | | |
| V4-28 | 276 | 0.02% | | IGHD4-23*01 | 4766 | 0.44% | | | | |
| Vl-58 | 268 | 0.02% | | IGHD2-8*02 | 4731 | 0.44% | | | | |
| V1-3 | 19 | 0.00% | | IGHD3-16*02 | 3661 | 0.34% | | | | |
| V3-35 | 12 | 0.00% | | IGHD5-24*01 | 3324 | 0.31% | | | | |
| V7-4-1 | 11 | 0.00% | | IGHD4-11*01 | 2911 | 0.27% | | | | |
| | | | | IGHD2-8*01 | 1910 | 0.18% | | | | |
| | | | | IGHD1/OR15-1a*01 | 1623 | 0.15% | | | | |
| | | | | IGHD2/OR15-2a*01 | 1374 | 0.13% | | | | |
| | | | | IGHD6-25*01 | 1299 | 0.12% | | | | |
| | | | | IGHD2-2*03 | 1242 | 0.11% | | | | |
| | | | | IGHD5/OR15-5a*01 | 1099 | 0.10% | | | | |
| | | | | IGHD2-2*02 | 932 | 0.09% | | | | |
| | | | | IGHD3-10*02 | 379 | 0.03% | | | | |
| | | | | IGHD3-16*03 | 269 | 0.02% | | | | |
| | | | | IGHD5-18*02 | 109 | 0.01% | | | | |
| | | | | IGHD3-3*02 | 66 | 0.01% | | | | |

Likewise, of the 1239828 valid sequencing Reads from the immunized IgM-KL-hIgHD mouse samples, the expression of most VH gene fragments, all DH genes, and all JH gene fragments were detected (Table 21).

**Table 21**

| IgHV gene | Count | Ratio | | IgHD gene | Count | Ratio | | IgHJ gene | Count | Ratio |
|---|---|---|---|---|---|---|---|---|---|---|
| V5-51 | 428570 | 34.57% | | IGHD7-27*01 | 351325 | 35.61% | | IGHJ3*02 | 722361 | 58.26% |
| V4-39 | 229955 | 18.55% | | IGHD3-16*01 | 111405 | 11.29% | | IGHJ4*01 | 371538 | 29.97% |
| V4-34 | 182929 | 14.75% | | IGHD6-19*01 | 77868 | 7.89% | | IGHJ2*01 | 42795 | 3.45% |
| V1-24 | 99824 | 8.05% | | IGHD3-10*01 | 64145 | 6.50% | | IGHJ6*01 | 42316 | 3.41% |
| V1-2 | 81978 | 6.61% | | IGHD6-13*01 | 44100 | 4.47% | | IGHJ5*02 | 27400 | 2.21% |
| V3-48 | 58599 | 4.73% | | IGHD2-15*01 | 40594 | 4.11% | | IGHJ1*01 | 16467 | 1.33% |
| V3-23 | 42082 | 3.39% | | IGHD1-26*01 | 39785 | 4.03% | | IGHJ5*01 | 10359 | 0.84% |
| V6-1 | 33347 | 2.69% | | IGHD4-23*01 | 31545 | 3.20% | | IGHJ3*01 | 6549 | 0.53% |
| V4-59 | 15064 | 1.22% | | IGHD1-7*01 | 30717 | 3.11% | | IGHJ6*04 | 43 | 0.00% |
| V3-21 | 11515 | 0.93% | | IGHD5-12*01 | 24819 | 2.52% | | | | |
| V4-4 | 10261 | 0.83% | | IGHD1-1*01 | 21389 | 2.17% | | | | |
| V3-30 | 8966 | 0.72% | | IGHD3-22*01 | 20835 | 2.11% | | | | |
| V3-33 | 8208 | 0.66% | | IGHD3/OR15-3a*01 | 15847 | 1.61% | | | | |
| V3-7 | 4995 | 0.40% | | IGHD6-6*01 | 15225 | 1.54% | | | | |
| V3-43 | 4558 | 0.37% | | IGHD1-20*01 | 15066 | 1.53% | | | | |
| V3-53 | 4476 | 0.36% | | IGHD5-18*01 | 8238 | 0.84% | | | | |
| V3-11 | 4462 | 0.36% | | IGHD3-10*03 | 7953 | 0.81% | | | | |
| V4-30-2 | 2837 | 0.23% | | IGHD3-3*01 | 7732 | 0.78% | | | | |
| V5-10-1 | 2179 | 0.18% | | IGHD3-9*01 | 6640 | 0.67% | | | | |
| V3-49 | 1570 | 0.13% | | IGHD4/OR15-4a*01 | 6025 | 0.61% | | | | |
| V3-15 | 1497 | 0.12% | | IGHD2-8*01 | 5257 | 0.53% | | | | |
| V1-18 | 611 | 0.05% | | IGHD2-21*01 | 5167 | 0.52% | | | | |
| V1-46 | 564 | 0.05% | | IGHD2-21*02 | 5007 | 0.51% | | | | |
| V3-13 | 330 | 0.03% | | IGHD1-14*01 | 4865 | 0.49% | | | | |
| V1-58 | 249 | 0.02% | | IGHD2-2*01 | 4763 | 0.48% | | | | |
| V4-28 | 152 | 0.01% | | IGHD4-17*01 | 3848 | 0.39% | | | | |
| V3-20 | 12 | 0.00% | | IGHD3-16*02 | 3101 | 0.31% | | | | |
| V3-35 | 9 | 0.00% | | IGHD4-11*01 | 2624 | 0.27% | | | | |
| V3-64D | 9 | 0.00% | | IGHD2-8*02 | 2307 | 0.23% | | | | |
| V1-3 | 8 | 0.00% | | IGHD6-25*01 | 2062 | 0.21% | | | | |
| V2-26 | 8 | 0.00% | | IGHD5-24*01 | 1965 | 0.20% | | | | |
| V2-5 | 4 | 0.00% | | IGHD2/OR15-2a*01 | 1398 | 0.14% | | | | |
| | | | | IGHD1/OR15-1a*01 | 946 | 0.0009589 | | | | |
| | | | | IGHD2-2*03 | 552 | 0.06% | | | | |
| | | | | IGHD5/OR15-5a*01 | 397 | 0.04% | | | | |
| | | | | IGHD2-2*02 | 394 | 0.04% | | | | |
| | | | | IGHD3-10*02 | 359 | 0.04% | | | | |
| | | | | IGHD3-16*03 | 148 | 0.02% | | | | |
| | | | | IGHD5-18*02 | 71 | 0.01% | | | | |
| | | | | IGHD3-3*02 | 67 | 0.01% | | | | |

Comparison of Table 20 with Table 21 showed that the proportions of some VH, DH, and J H genes were significantly up-regulated or decreased before and after the immunization, indicating that the humanized nanobody-producing mouse had a good response to antigen immunity.

### Example 13: Phenotype detection of IgA-KL-hIgHD homozygous mice

### (I) Antigen immune response and titer detection of IgA-KL-hIgHD homozygous mice

IgA-KL-hIgHD homozygous mice and IgA-KL mice were immunized with the CD93 protein. Specifically, mice aged 6-8 weeks were selected. The antigen was mixed with an equal volume of Freund's complete adjuvant and emulsified until it no longer dissolved in water. Then the emulsion can be injected subcutaneously at multiple sites into the mice. For the primary immunization, the injection dose was 100 µg per mouse and the injection volume was 200 µL per mouse.

Two weeks after the primary immunization, a second immunization was carried out subcutaneously. The antigen was emulsified with an equal volume of Freund's incomplete adjuvant and then injected subcutaneously at multiple sites into the mice. The injection dose was reduced to 50 µg per mouse and the injection volume was 200 µL per mouse. The specific immunization scheme was shown in Table 22.

**Table 22 Immunization scheme for IgA-KL-hIgHD mice and IgA-KL mice**

| **IgA-KL-hIgHD: #3576,#3577** | **IgA-KL: #3702, #3706** | **immunization** | **Time** | **Notes** |
|---|---|---|---|---|
| 100µg CD93 /200 µL/animal (Freund's complete adjuvant) | | Primary immunization | 2023-03-03 | subcutaneously at multiple points |
| 50µg CD93/200µL/animal (Freund's incomplete adjuvant) | | Second immunization | 3/17/2023 | subcutaneously at multiple points |
| 50µg CD93/200µL/animal (Freund's incomplete adjuvant) | | Third immunization | 3/31/2023 | subcutaneously at multiple points |
| 50µg CD93/200µL/animal (Freund's incomplete adjuvant) | | Forth immunization | 4/7/2023 | subcutaneously at multiple points |
| | | | 4/14/2023 | The serum was taken and subjected to titer detection by ELISA. |
| 50µg CD93/200µL/animal (Freund's incomplete adjuvant) | | Fifth immunization | 4/14/2023 | subcutaneously at multiple points |
| | | | 4/21/2023 | The serum was taken and subjected to titer detection by ELISA. |

The serum titer detection was performed as follows: the antigen protein was diluted to 2 µg/mL, 100 µL of the dilution was taken and added to a polystyrene enzyme-linked plate for plating, and the humanized nanobody that specifically binds to the antigen protein in sera of IgA-KL-hIgHD mice and IgA-KL mice was detected using goat anti-mouse IgG Fc HRP (JACKSON, 115-035-071) antibody.

As shown in Figure 28, blood samples were collected on the 7th day after the fifth immunization with the antigenic protein, and ELISA assays for serum titers showed that antibodies specifically binding to the antigen protein were detected in IgA-KL-hIgHD homozygous mice, and the serum titers in all cases exceeded 1:72000. In contrast, the IgA-KL background mice had no immune titer, indicating that the antibodies found in the IgA-KL-hIgHD mice were produced by the expression of the introduced human antibody gene sequence hIgHD.

### (II) Western Blot assay of serum from IgA-KL-hIgHD homozygous mice immunized with CD93

Sera from IgA-KL-hIgHD mice (# 3756,#3577), IgA- KL(#3702, #3706) after the fifth immunization with CD93 and unimmunized C57BL/6 mice (#10252) were taken for Western assay to verify whether human-mouse chimeric nanobodies were expressed in IgA-KL-hIgHD homozygous mice.

The loading volume of the serum samples in each group of tests was 0.2 µL. After boiling for denaturation, the samples were loaded onto a 12% SDS-PAGE gel for electrophoresis, and then transferred onto a PVDF membrane and blocked, goat anti-mouse IgG Fc HRP (JACKSON, 115-035-071) was used to detect the expression of human-mouse chimeric nanobodies in the mouse serum as described above. The results were shown in Table 29 below.

As seen from Figure 29, human-mouse chimeric nanobodies were detected in IgA-KL-hIgHD (#3756, and #3577) immunized with CD93 antigen, and IgG antibodies were not detected in the CD93 immunized IgA-KL (#3702, #3706), and the intact mouse antibody proteins were detected in the unimmunized C57BL/6 mice (#10252), demonstrating that human-mouse chimeric nanobodies were expressed in IgA-KL-hIgHD mice.

(III) Human variable region gene V(D)J rearrangement in IgA-KL-hIgHD homozygous mice 1) NGS sequencing analysis of sequences of mouse heavy chain variable region genes One unimmunized mouse and one CD93-immunized IgA-KL-hIgHD homozygous mouse were selected, and splenocytes from the mice were taken for extracting RNA, and the RNA was then reverse transcribed into cDNA using a reverse transcription kit (Takara, 6110A). PCR amplification was performed by using the combination of 10 primers for human V genes with three specific primers for human J genes, respectively, with the cDNA of the sample as a template, to obtain the heavy chain variable region fragments which were then subjected to subsequent sequencing. The sequences of the human gene specific primers were shown in Table 19 above. Since the PCR products of the two samples required NGS sequencing and data analysis, specific barcode sequences needed to be added at the 5' end of primers in Table 19. Therefore, ACAGAG was added to the 5' end of the primer F, and AGACTG was added to the 5 'end of the primer R used for the IgA-KL-hIgHD unimmunized mice; AGTGCT was added to the 5 'end of the primer F and TCCGGA was added to the 5' end of the primer R used for the IgA KL-hIgHD immunized mice. The samples of IgA-KL-hIgHD were amplified by PCR using primers added with barcode sequences, and the PCR amplification products were subjected to agarose gel electrophoresis. The results were shown in Figures 30A and 30B, which represent the PCR products from the unimmunized IgA-KL-hIgHD mouse samples and the PCR products from the immunized IgA-KL-hIgHD mouse samples, respectively.

Next, the PCR products were subjected to NGS sequencing.

### 2) Analysis of NGS sequencing results

The sequencing results and human immunoglobulin sequences were analyzed by bioinformatics technology to identify the expression of human VH, DH, JH genes after V(D)J recombination. As a result, of the 895880 valid sequencing Reads from the unimmunized IgA-KL-hIgHD mouse samples, the expression of most VH gene fragments, all DH genes, and all JH gene fragments were detected (Table 23). Among these gene fragments, some VH genes were located near the constant region, while others were farther from it. The data of Table 23 indicated that human VH, DH, and JH genes on the human-mouse chimeric nanobody genes transferred by the hIgHD scheme can be rearranged and expressed in IgM-KL background mice.

**Table 23**

| IgHV gene | Count | Ratio | | IgHD gene | Count | Ratio | | IgHJ gene | Count | Ratio |
|---|---|---|---|---|---|---|---|---|---|---|
| V4-34 | 229652 | 25.63 % | | IGHD7-27*01 | 248290 | 31.15% | | IGHJ3*02 | 399954 | 44.64% |
| V5-51 | 161159 | 17.99% | | IGHD3-16*01 | 101873 | 12.78% | | IGHJ4*01 | 310127 | 34.62% |
| V3-48 | 103926 | 11.60% | | IGHD3-10*01 | 70668 | 8.87% | | IGHJ2*01 | 76012 | 8.48% |
| V1-2 | 103564 | 11.56% | | IGHD1-26*01 | 61042 | 7.66% | | IGHJ6*01 | 46306 | 5.17% |
| V4-39 | 70070 | 7.82% | | IGHD6-19*01 | 46059 | 5.78% | | IGHJ5*02 | 35551 | 3.97% |
| V4-59 | 44256 | 4.94% | | 1GHD1-1*01 | 37266 | 4.68% | | IGHJ3*01 | 10467 | 1.17% |
| V3-23 | 33070 | 3.69% | | IGHD6-13*01 | 27137 | 3.41% | | IGHJ5*01 | 9131 | 1.02% |
| V1-24 | 31697 | 3.54% | | IGHD2-15*01 | 21678 | 2.72% | | IGHJ1*01 | 8279 | 0.92% |
| V3-30 | 20809 | 2.32% | | IGHD1-7*01 | 21370 | 2.68% | | IGHJ6*04 | 53 | 0.01% |
| V3-33 | 17849 | 1.99% | | IGHD6-6*01 | 18521 | 2.32% | | | | |
| V3-21 | 12757 | 1.42% | | IGHD3/OR15-3a*01 | 15646 | 1.96% | | | | |
| V3-53 | 11669 | 1.30% | | IGHD5-18*01 | 11426 | 1.43% | | | | |
| V4-4 | 9310 | 1.04% | | IGHD3-9*01 | 11025 | 1.38% | | | | |
| V6-1 | 9033 | 1.01% | | IGHD1-20*01 | 9879 | 1.24% | | | | |
| V3-43 | 8208 | 0.92% | | IGHD3-10*03 | 8453 | 1.06% | | | | |
| V3-49 | 6226 | 0.69% | | IGHD6-25*01 | 8137 | 1.02% | | | | |
| V4-30-2 | 5239 | 0.58% | | IGHD2-2*01 | 6866 | 0.86% | | | | |
| V3-7 | 4678 | 0.52% | | IGHD2-21*02 | 6672 | 0.84% | | | | |
| V3-11 | 4356 | 0.49% | | IGHD4/OR15-4a*01 | 6365 | 0.80% | | | | |
| V1-46 | 2576 | 0.29% | | IGHD1-14*01 | 6296 | 0.79% | | | | |
| V1-18 | 1970 | 0.22% | | IGHD3-3*01 | 6281 | 0.79% | | | | |
| V3-15 | 1336 | 0.15% | | IGHD4-23*01 | 5242 | 0.66% | | | | |
| V3-13 | 1159 | 0.13% | | IGHD4-17*01 | 4879 | 0.61% | | | | |
| V5-10-1 | 564 | 0.06% | | IGHD5-12*01 | 4743 | 0.60% | | | | |
| V4-28 | 333 | 0.04% | | IGHD3-22*01 | 4620 | 0.58% | | | | |
| V1-58 | 279 | 0.03% | | IGHD2-21*01 | 4559 | 0.57% | | | | |
| V3-20 | 69 | 0.01% | | IGHD2-8*01 | 3841 | 0.48% | | | | |
| V3-35 | 25 | 0.00% | | IGHD2/OR15-2a*01 | 3217 | 0.40% | | | | |
| V3-64D | 14 | 0.00% | | IGHD2-8*02 | 3018 | 0.38% | | | | |
| V1-3 | 12 | 0.00% | | IGHD3-16*02 | 2783 | 0.35% | | | | |
| V2-26 | 10 | 0.00% | | IGHD4-11*01 | 2577 | 0.32% | | | | |
| V7-4-1 | 4 | 0.00% | | IGHD5-24*01 | 1613 | 0.20% | | | | |
| V1-45 | 1 | 0.00% | | IGHD2-2*02 | 1269 | 0.16% | | | | |
| | | | | IGHD3-10*02 | 1136 | 0.14% | | | | |
| | | | | IGHD1/OR15-1a*01 | 914 | 0.11% | | | | |
| | | | | IGHD5/OR15-5a*01 | 813 | 0.10% | | | | |
| | | | | IGHD2-2*03 | 292 | 0.04% | | | | |
| | | | | IGHD3-16*03 | 290 | 0.04% | | | | |
| | | | | IGHD3-3*02 | 119 | 0.01% | | | | |
| | | | | IGHD5-18*02 | 93 | 0.01% | | | | |

Likewise, of the 877915 valid sequencing Reads from the immunized IgA-KL-hIgHD mouse samples, the expression of most VH gene fragments, all DH genes, and all JH gene fragments was detected (Table 24).

**Table 24**

| IgHV gene | Count | Ratio | | IgHD gene | Count | Ratio | | IgHJ gene | Count | Ratio |
|---|---|---|---|---|---|---|---|---|---|---|
| V5-51 | 209927 | 23.91% | | IGHD7-27*01 | 224970 | 28.71% | | IGHJ3*02 | 369433 | 42.08% |
| V4-39 | 144071 | 16.41% | | IGHD6-19*01 | 183870 | 23.47% | | IGHJ4*01 | 256703 | 29.24% |
| V3-33 | 113796 | 12.96% | | IGHD3-16*01 | 52480 | 6.70% | | IGHJ6*01 | 149180 | 16.99% |
| V4-34 | 98237 | 11.19% | | IGHD3-10*01 | 49057 | 6.26% | | IGHJ2*01 | 54318 | 6.19% |
| V1-2 | 86629 | 9.87% | | IGHD1-26*01 | 41992 | 5.36% | | IGHJ5*02 | 15556 | 1.77% |
| V3-48 | 64696 | 7.37% | | IGHD2-15*01 | 39895 | 5.09% | | IGHJ1*01 | 14329 | 1.63% |
| V1-24 | 41274 | 4.70% | | IGHD1-1*01 | 33403 | 4.26% | | IGHJ3*01 | 10794 | 1.23% |
| V4-59 | 23567 | 2.68% | | IGHD6-13*01 | 23537 | 3.00% | | IGHJ5*01 | 7497 | 0.85% |
| V3-23 | 20696 | 2.36% | | IGHD1-7*01 | 15615 | 1.99% | | IGHJ6*04 | 105 | 0.01% |
| V3-21 | 13208 | 1.50% | | IGHD3-10*03 | 12998 | 1.66% | | | | |
| V6-1 | 13089 | 1.49% | | IGHD6-6*01 | 10253 | 1.31% | | | | |
| V3-30 | 12569 | 1.43% | | IGHD3/OR15-3a*01 | 8904 | 1.14% | | | | |
| V4-30-2 | 8691 | 0.99% | | IGHD2-2*01 | 7984 | 1.02% | | | | |
| V4-4 | 6958 | 0.79% | | IGHD1-20*01 | 7514 | 0.96% | | | | |
| V3-43 | 4338 | 0.49% | | IGHD4-23*01 | 6347 | 0.81% | | | | |
| V3-53 | 3666 | 0.42% | | IGHD4-17*01 | 6192 | 0.79% | | | | |
| V3-49 | 3274 | 0.37% | | IGHD4/OR15-4a*01 | 6176 | 0.79% | | | | |
| V3-7 | 2203 | 0.25% | | IGHD3-22*01 | 5487 | 0.70% | | | | |
| V3-11 | 1832 | 0.21% | | IGHD3-9*01 | 4959 | 0.63% | | | | |
| V1-18 | 1134 | 0.13% | | IGHD2-8*02 | 3815 | 0.49% | | | | |
| V3-15 | 1010 | 0.12% | | IGHD5-18*01 | 3799 | 0.48% | | | | |
| V1-46 | 951 | 0.11% | | IGHD1-14*01 | 3768 | 0.48% | | | | |
| V3-13 | 725 | 0.08% | | IGHD2-2*02 | 3651 | 0.47% | | | | |
| V5-10-1 | 671 | 0.08% | | IGHD2-21*01 | 3433 | 0.44% | | | | |
| V4-28 | 336 | 0.04% | | IGHD4-11*01 | 3234 | 0.41% | | | | |
| V1-58 | 304 | 0.03% | | IGHD5-12*01 | 3129 | 0.40% | | | | |
| V3-20 | 23 | 0.00% | | IGHD2-21*02 | 2886 | 0.37% | | | | |
| V1-3 | 18 | 0.00% | | IGHD3-3*01 | 2832 | 0.36% | | | | |
| V3-64D | 10 | 0.00% | | IGHD2-8*01 | 2621 | 0.33% | | | | |
| V7-4-1 | 5 | 0.00% | | IGHD5-24*01 | 2041 | 0.26% | | | | |
| V2-5 | 5 | 0.00% | | IGHD3-16*02 | 1481 | 0.19% | | | | |
| V3-35 | 2 | 0.00% | | IGHD2/OR15-2a*01 | 1473 | 0.19% | | | | |
| | | | | IGHD1/OR15-1a*01 | 890 | 0.11% | | | | |
| | | | | IGHD6-25*01 | 882 | 0.11% | | | | |
| | | | | IGHD3-10*02 | 569 | 0.07% | | | | |
| | | | | IGHD5/OR15-5a*01 | 444 | 0.06% | | | | |
| | | | | IGHD3-16*03 | 356 | 0.05% | | | | |
| | | | | IGHD2-2*03 | 285 | 0.04% | | | | |
| | | | | IGHD3-3*02 | 130 | 0.02% | | | | |
| | | | | IGHD5-18*02 | 113 | 0.01% | | | | |

Comparison of Table 23 with Table 24 showed that the proportions of some VH, DH, and J H genes were significantly up-regulated or decreased before and after the immunization, indicating that the humanized nanobody-producing mouse had a good response to antigen immunity.

Finally, it should be noted that the above embodiments are only used to illustrate the technical solutions of the present invention and not to limit it; although the present invention has been described in detail with reference to the foregoing embodiments, it will be understood by one of ordinary skill in the art that the technical solutions described in the foregoing embodiments can still be modified or some technical features can be equivalently substituted; however, these modifications or substitutions do not make the essence of the corresponding technical solutions departing from the spirit and scope of the technical solutions of various embodiments of the present invention.

## Claims

1. A method for preparing a genetically modified non-human mammal, which comprises the following steps:
bringing about a disruption of an endogenous heavy chain immunoglobulin gene loci in a non-human mammal.

2. The method of claim 1, wherein the non-human mammal is a mouse, and the disruption of the endogenous heavy chain immunoglobulin gene loci include deletions of the following gene fragments:
a CH1 fragment of mouse antibody gene heavy chain IgHM, mouse antibody gene light chain Igxc, and a fragment between IgLc2 and IgLc1 of mouse antibody gene light chain Igλ;
preferably, wherein the deletions of the gene fragments are performed through CRISPR-Cas9 gene editing technique;
wherein the sgRNA for deleting the CH1 fragment of mouse antibody gene heavy chain IgHM comprises a sgRNA as shown in SEQ ID NO: 1 and a sgRNA as shown in SEQ ID NO: 2; and/or,
the sgRNA for deleting the mouse antibody gene light chain Igκc comprises a sgRNA as shown in SEQ ID NO: 3; and/or, the sgRNA for deleting the fragment between IgLc2 and IgLc1 of mouse antibody gene light chain Igλ comprises a sgRNA as shown in SEQ ID NO: 4 and a sgRNA as shown in SEQ ID NO: 5.

3. The method of claim 1, wherein the non-human mammal is a mouse, and the disruption of the endogenous heavy chain immunoglobulin gene loci include deletions of the following gene fragments:
a fragment between IgHM and IgHA-CH1 of mouse antibody gene heavy chain, mouse antibody gene light chain Igxc, and a fragment between IgLc2 and IgLc1 of mouse antibody gene light chain Igλ;
preferably, wherein the deletions of the gene fragments are performed through CRISPR-Cas9 gene editing technique;
wherein the sgRNA for deleting the fragment between IgHM and IgHA-CH1 of mouse antibody gene heavy chain comprises a sgRNA as shown in SEQ ID NO: 1 and a sgRNA as shown in SEQ ID NO: 6; and/or, the sgRNA for deleting the mouse antibody gene light chain Igκc comprises a sgRNA as shown in SEQ ID NO: 3; and/or the sgRNA for deleting a fragment between IgLc2 and IgLc1 of mouse antibody gene light chain Igλ comprises a sgRNA as shown in SEQ ID NO: 4 and a sgRNA as shown in SEQ ID NO: 5.

4. A method for preparing a genetically modified non-human mammal, which comprises the following steps:
(1) preparing a genetically modified non-human mammal I according to the method of any one of claims 1-3; and
(2) introducing a human IGHV gene, a human IGHD gene, a human IGHJ gene as well as an endogenous IgHG2c gene, IgHE gene, IgHA gene, and LCR region of the non-human mammal into the genetically modified non-human mammal I obtained in step (1).

5. The method of claim 4, wherein the non-human mammal is a mouse, and in step (2),
the human IGHV gene includes at least the following 18 human IGHV genes:
hIGHV4-59, hIGHV3-53, hIGHV5-51, hIGHV3-49, hIGHV3-48, hIGHV1-46, hIGHV3-43, hIGHV4-39, hIGHV3-33, hIGHV4-30-2, hIGHV1-24, hIGHV3-23, hIGHV3-21, hIGHV3-11, hIGHV3-7, hIGHV4-4, hIGHV1-2, hIGHV6-1; optionally, the human IGHV gene further include one or several or all of the human IGHV genes selected from: hIGHV1-58, hIGHV1-45, hIGHV3-35, hIGHV4-28, hIGHV2-26, hIGHV3-20, hIGHV1-18, hIGHV3-15, hIGHV3-13, hIGHV5-10-1, hIGHV3-64D, hIGHV2-5, hIGHV7-4-1, hIGHV1-3; and further optionally, the human IGHV gene further include one or several or all of the human IGHV genes selected from: hIGHV3-60, hIGHV3-57, hIGHV7-56, hIGHV4-55, hIGHV3-54, hIGHV3-52, hIGHV3-50, hIGHV3-47, hIGHV3-42, hIGHV3-41, hIGHV3-38, hIGHV3-37, hIGHV3-36, hIGHV7-34-1, hIGHV4-34, hIGHV3-33-2, hIGHV3-32, hIGHV3-30-2, hIGHV3-30, hIGHV3-29, hIGHV7-27, hIGHV3-25, hIGHV3-22, hIGHV3-19, hIGHV1-17, hIGHV3-16, hIGHV1-14, hIGHV1-12, hIGHV3-6;
and/or, the human IGHD gene includes at least the following 15 human IGHD genes:
IGHD1-1, IGHD3-3, IGHD6-6, IGHD1-7, IGHD3-10, IGHD6-13, IGHD1-14, IGHD2-15, IGHD3-16, IGHD5-18, IGHD6-19, IGHD1-20, IGHD3-22, IGHD1-26, IGHD7-27; optionally, the human IGHD gene further includes one or several or all of the human IGHD genes selected from: IGHD2-2, IGHD2-8, IGHD3-9, IGHD3-10, IGHD4-11, IGHD5-12, IGHD3-16, IGHD4-17, IGHD2-21, IGHD4-23, IGHD5-24, IGHD6-25; and further optionally, the human IGHD gene further includes one or several or all of the human IGHD genes selected from: IGHD3-3, IGHD3-10, IGHD3-16, IGHD5-18;
and/or, the human IGHJ gene includes all the human IGHJ genes;
and/or, the endogenous IgHG2c gene of the non-human mammal is the endogenous complete IgHG2c gene, or the endogenous IgHG2c gene segment without the CH1 domain.

6. The method of claim 5, wherein in step (2), the human IGHV gene, the human IGHD gene, the human IGHJ gene as well as the endogenous IgHG2c gene, IgHE gene, IgHA gene, and LCR region of the non-human mammal are introduced by the following method:
introducing the following materials into the mouse obtained in step (1):
(I) n BAC clones that comprise all of the human IGHV genes, the human IGHD genes, and the human IGHJ genes, in total; wherein n is an integer between 3 and 8, preferably is an integer between 4 and 7, and more preferably is 4; each of the n BAC clones has a 5 kb to 50 kb of gene fragment at the beginning or the end thereof, and for every two adjacent BAC clones, the 5 kb to 50 kb of gene fragment at the end of one BAC clone is homologous to that at the beginning of the other BAC clone, so that they can be interconnected with each other through the homologous gene sequences; and
(II) one or more BAC clones comprising the endogenous IgHG2c gene, IgHE gene, IgHA gene, and LCR region of the non-human mammal.

7. The method of claim 6, wherein in step (2), the human IGHV genes, the human IGHD genes, the human IGHJ genes as well as the endogenous IgHG2c gene, IgHE gene, IgHA gene, and LCR region of the non-human mammal are introduced by the following method:
introducing the following 6 BAC clones into the mouse obtained in step (1):
(i) 4 BAC clones containing all the human IGHV genes,
(ii) 1 BAC clone containing all the human IGHD genes and all the human IGHJ genes, and
(iii) 1 BAC clone containing the endogenous IgHG2c gene, IgHE gene, IgHA gene and LCR region of the non-human mammal;
wherein for BAC clones in (i), and for BAC clones in (i) and (ii), each BAC clone has a 5 kb to 50 kb and preferably a 5 kb to 20 kb of gene fragment at the beginning or the end thereof, and for every two adjacent BAC clones, the 5 kb to 50 kb and preferably the 5 kb to 20 kb of gene fragment at the end of one BAC clone is homologous to that at the beginning of the other BAC clone, so that they can be interconnected with each other through the homologous gene sequences;
preferably, the genes contained in the 6 BAC clones are shown in the table below:
| BAG Clones No. | CH17-268I9 | CTD-3054M17 | CTD-2548B8-CZ | RP11-965B13 | CH17-185P21-CZ | RP23-351J19 -CZ |
|---|---|---|---|---|---|---|
| Important sequence contained | hIGHV3-60 | hIGHV3-41 | hIGHV3-23 | hIGHV3-11 | hIGHV1-2 | mIgHG2c-CH1 |
| | hIGHV4-59 | hIGHV4-39 | hIGHV3-22 | hIGHV5-10-1 | hIGHV6-1 | mIgHE |
| | hIGHV1-58 | hIGHV3-38 | hIGHV3-21 | hIGHV3-64D | hIGHD1-1 | mIgHA |
| | IGHV3-57 | hIGHV3-37 | hIGHV3-20 | hIGHV3-7 | hIGHD2-2 | mLCR |
| | hIGHV7-56 | hIGHV3-36 | hIGHV3-19 | hIGHV3-6 | hIGHD3-3 | |
| | hIGHV4-55 | hIGHV3-35 | hIGHV1-18 | hIGHV2-5 | hIGHD4-4 | |
| | hIGHV3-54 | hIGHV7-34-1 | hIGHV1-17 | hIGHV7-4-1 | hIGHD5-5 | |
| | hIGHV3-53 | hIGHV4-34 | hIGHV3-16 | hIGHV4-4 | hIGHD6-6 | |
| | hIGHV3-52 | hIGHV3-33-2 | hIGHV3-15 | hIGHV1-3 | HIGHD1-7 | |
| | hIGHV5-51 | hIGHV3-33 | hIGHV1-14 | hIGHV1-2 | hIGHD2-8 | |
| | hIGHV3-50 | hIGHV3-32 | hIGHV3-13 | hIGHV6-1 | hIGHD3-9 | |
| | hIGHV3-49 | hIGHV4-30-2 | hIGHV1-12 | | hIGHD3-10 | |
| | hIGHV3-48 | hIGHV3-30-2 | hIGHV3-11 | | hIGHD4-11 | |
| | hIGHV3-47 | IGHV3-30 | | | hIGHD5-12 | |
| | hIGHV1-46 | hIGHV3-29 | | | hIGHD6-13 | |
| | hIGHV1-45 | hIGHV4-28 | | | hIGHD1-14 | |
| | hIGHV3-43 | hIGHV7-27 | | | hIGHD2-15 | |
| | hIGHV3-42 | hIGHV2-26 | | | hIGHD3-16 | |
| | hIGHV3-41 | hIGHV3-25 | | | hIGHD4-17 | |
| | hIGHV4-39 | hIGHV1-24 | | | hIGHD5-18 | |
| | hIGHV3-38 | hIGHV3-23 | | | hIGHD6-19 | |
| | hIGHV3-37 | hIGHV3-22 | | | hIGHD1-20 | |
| | | | | | hIGHD2-21 | |
| | | | | | hIGHD3-22 | |
| | | | | | hIGHD4-23 | |
| | | | | | hIGHD5-24 | |
| | | | | | hIGHD6-25 | |
| | | | | | hIGHD1-26 | |
| | | | | | hIGHD7-27 | |
| | | | | | hIGHJ1P | |
| | | | | | hIGHJ1 | |
| | | | | | hIGHJ2 | |
| | | | | | hIGHJ2P | |
| | | | | | hIGHJ3 | |
| | | | | | hIGHJ4 | |
| | | | | | hIGHJ5 | |
| | | | | | hIGHJ3P | |
| | | | | | hIGHJ6 | |
wherein "mIgHG2c-CH1" represented the mIgHG2c gene sequence without the CH1 domain.

8. The method of claim 7, wherein the human IGHV genes, the human IGHD genes, and the human IGHJ genes are operatively linked to each other and subjected to VDJ-rearrangement, and the operatively linked and/or VDJ-rearranged human IGHV genes, the human IGHD genes, and the human IGHJ genes, are operatively linked to the endogenous IgHG2c gene, IgHE gene, IgHA gene, and LCR region of the non-human mammal;
and/or, there is a Switch region of the endogenous IgHM of the non-human mammal between the human IGHJ gene and the endogenous IgHG2c gene of the non-human mammal.

9. A genetically modified non-human mammal, which comprises a disruption in an endogenous heavy chain immunoglobulin gene loci, and the endogenous heavy chain immunoglobulin gene loci comprises a human IGHV gene, a human IGHD gene, a human IGHJ gene as well as an endogenous IgHG2c gene, IgHE gene, IgHA gene, and LCR region of the non-human mammal.

10. The genetically modified non-human mammal according to claim 9, wherein the disruption in the endogenous heavy chain immunoglobulin gene loci includes deletions of the following gene fragments:
a CH1 fragment of mouse antibody gene heavy chain IgHM, mouse antibody gene light chain Igκc, and a fragment between IgLc2 and IgLc1 of mouse antibody gene light chain Igλ;
and/or, the human IGHV gene includes at least the following 18 human IGHV genes: hIGHV4-59, hIGHV3-53, hIGHV5-51, hIGHV3-49, hIGHV3-48, hIGHV1-46, hIGHV3-43, hIGHV4-39, hIGHV3-33, hIGHV4-30-2, hIGHV1-24, hIGHV3-23, hIGHV3-21, hIGHV3-11, hIGHV3-7, hIGHV4-4, hIGHV1-2, and hIGHV6-1; preferably, the human IGHV gene further includes one or several or all of the human IGHV genes selected from: hIGHV1-58, hIGHV1-45, hIGHV3-35, hIGHV4-28, hIGHV2-26, hIGHV3-20, hIGHV1-18, hIGHV3-15, hIGHV3-13, hIGHV5-10-1, hIGHV3-64D, hIGHV2-5, hIGHV7-4-1, and hIGHV1-3; further preferably, the human IGHV gene further includes one or several or all of the human IGHV genes selected from: hIGHV3-60, hIGHV3-57, hIGHV7-56, hIGHV4-55, hIGHV3-54, hIGHV3-52, hIGHV3-50, hIGHV3-47, hIGHV3-42, hIGHV3-41, hIGHV3-38, hIGHV3-37, hIGHV3-36, hIGHV7-34-1, hIGHV4-34, hIGHV3-33-2, hIGHV3-32, hIGHV3-30-2, hIGHV3-30, hIGHV3-29, hIGHV7-27, hIGHV3-25, hIGHV3-22, hIGHV3-19, hIGHV1-17, hIGHV3-16, hIGHV1-14, hIGHV1-12, and hIGHV3-6;
and/or, the human IGHD gene includes at least the following 15 human IGHD genes: IGHD1-1, IGHD3-3, IGHD6-6, IGHD1-7, IGHD3-10, IGHD6-13, IGHD1-14, IGHD2-15, IGHD3-16, IGHD5-18, IGHD6-19, IGHD1-20, IGHD3-22, IGHD1-26, and IGHD7-27; preferably, the human IGHD gene further includes one or several or all of the human IGHD genes selected from: IGHD2-2, IGHD2-8, IGHD3-9, IGHD3-10, IGHD4-11, IGHD5-12, IGHD3-16, IGHD4-17, IGHD2-21, IGHD4-23, IGHD5-24, and IGHD6-25; further preferably, the human IGHD gene further includes one or several or all of the human IGHD genes selected from: IGHD3-3, IGHD3-10, IGHD3-16, and IGHD5-18;
and/or, the human IGHJ gene includes all the human IGHJ genes;
and/or, the endogenous IgHG2c gene of the non-human mammal is the complete endogenous IgHG2c gene, or the endogenous IgHG2c gene fragment without the CH1 domain;
preferably, the human IGHV genes, the human IGHD genes, and the human IGHJ genes are operatively linked to each other and subjected to VDJ-rearrangement; further preferably, the operatively linked and/or VDJ-rearranged human IGHV genes, human IGHD genes, and human IGHJ genes, are operatively linked to the endogenous IgHG2c gene, IgHE gene, IgHA gene, and LCR region of the non-human mammal;
preferably, there is a Switch region of the endogenous IgHM of the non-human mammal between the human IGHJ gene and the endogenous IgHG2c gene of the non-human mammal;
most preferably, the endogenous heavy chain immunoglobulin gene loci of the non-human mammal comprises all the genes shown in Table 13.

11. The genetically modified non-human mammal according to claim 9, wherein the disruption in the endogenous heavy chain immunoglobulin gene loci includes deletions of the following gene fragments:
a fragment between IgHM and IgHA-CH1 of mouse antibody gene heavy chain, mouse antibody gene light chain Igκc, and a fragment between IgLc2 and IgLc1 of mouse antibody gene light chain Igλ;
and/or, the human IGHV gene includes at least the following 18 human IGHV genes: hIGHV4-59, hIGHV3-53, hIGHV5-51, hIGHV3-49, hIGHV3-48, hIGHV1-46, hIGHV3-43, hIGHV4-39, hIGHV3-33, hIGHV4-30-2, hIGHV1-24, hIGHV3-23, hIGHV3-21, hIGHV3-11, hIGHV3-7, hIGHV4-4, hIGHV1-2, and hIGHV6-1; preferably, the human IGHV gene further includes one or several or all of the human IGHV genes selected from: hIGHV1-58, hIGHV1-45, hIGHV3-35, hIGHV4-28, hIGHV2-26, hIGHV3-20, hIGHV1-18, hIGHV3-15, hIGHV3-13, hIGHV5-10-1, hIGHV3-64D, hIGHV2-5, hIGHV7-4-1, and hIGHV1-3; further preferably, the human IGHV gene further includes one or several or all of the human IGHV genes selected from: hIGHV3-60, hIGHV3-57, hIGHV7-56, hIGHV4-55, hIGHV3-54, hIGHV3-52, hIGHV3-50, hIGHV3-47, hIGHV3-42, hIGHV3-41, hIGHV3-38, hIGHV3-37, hIGHV3-36, hIGHV7-34-1, hIGHV4-34, hIGHV3-33-2, hIGHV3-32, hIGHV3-30-2, hIGHV3-30, hIGHV3-29, hIGHV7-27, hIGHV3-25, hIGHV3-22, hIGHV3-19, hIGHV1-17, hIGHV3-16, hIGHV1-14, hIGHV1-12, and hIGHV3-6; and/or, the human IGHD gene includes at least the following 15 human IGHD genes: IGHD1-1, IGHD3-3, IGHD6-6, IGHD1-7, IGHD3-10, IGHD6-13, IGHD1-14, IGHD2-15, IGHD3-16, IGHD5-18, IGHD6-19, IGHD1-20, IGHD3-22, IGHD1-26, and IGHD7-27; preferably, the human IGHD gene further includes one or several or all of the human IGHD genes selected from: IGHD2-2, IGHD2-8, IGHD3-9, IGHD3-10, IGHD4-11, IGHD5-12, IGHD3-16, IGHD4-17, IGHD2-21, IGHD4-23, IGHD5-24, and IGHD6-25; further preferably, the human IGHD gene further includes one or several or all of the human IGHD genes selected from: IGHD3-3, IGHD3-10, IGHD3-16, and IGHD5-18;
and/or, the human IGHJ gene includes all the human IGHJ genes;
and/or, the endogenous IgHG2c gene of the non-human mammal is the complete endogenous IgHG2c gene, or the endogenous IgHG2c gene fragment without the CH1 domain;
preferably, the human IGHV genes, the human IGHD genes, and the human IGHJ genes are operatively linked to each other and subjected to VDJ-rearrangement; further preferably, the operatively linked and/or VDJ-rearranged human IGHV genes, human IGHD genes, and human IGHJ genes, are operatively linked to the endogenous IgHG2c gene, IgHE gene, IgHA gene, and LCR region of the non-human mammal;
preferably, there is a Switch region of the endogenous IgHM of the non-human mammal between the human IGHJ gene and the endogenous IgHG2c gene of the non-human mammal;
most preferably, the endogenous heavy chain immunoglobulin gene loci of the non-human mammal comprises all the genes shown in Table 13.

12. A method for preparing a humanized whole antibody or single heavy chain antibody or nanobody that specifically binds to an antigen, comprising:
(1) the genetically modified non-human mammal prepared by the method of any one of claims 4-8 or the genetically modified non-human mammal according to any one of claims 9-11 is exposed to an antigen;
(2) B cells are collected from the non-human mammal obtained in step (1), RNA is extracted and then reverse transcribed into cDNA, and antibody gene fragments are amplified using the cDNA as the template, and then are cloned into a phage display vector;
(3) the phage vector obtained in step (2) is used to express the target antibody; specifically, phages are subjected to panning to enrich for those expressing the target antibody, which are then induced to express the target antibody, i.e. a humanized whole antibody or single heavy chain antibody; and
Optionally, (4) the variable region fragments of the obtained single heavy chain antibody are obtained by cloning to obtain a humanized nanobody.

13. A method for preparing a humanized whole antibody or single heavy chain antibody or nanobody that specifically binds to an antigen, comprising:
(1) the genetically modified non-human mammal prepared by the method of any one of claims 4-8 or the genetically modified non-human mammal according to any one of claims 9-11 is exposed to an antigen, and then B cells are collected;
(2) nucleic acids encoding the immunoglobulin heavy chain variable region and optional the light chain variable region in B cells collected in step (1) are sequenced to obtain the nucleic acid sequences of a heavy chain variable region and a light chain variable region of a humanized monoclonal antibody or the nucleic acid sequence of a heavy chain variable region of a humanized nanobody,
(3) according to the sequences obtained in step (2), a humanized whole antibody or a humanized single heavy chain antibody that specifically binds to the antigen is obtained by expression; and Optionally, (4) the variable region fragments of the obtained single heavy chain antibody are obtained by cloning to obtain a humanized nanobody.

14. A sgRNA composition, which comprises: sgRNA for deleting CH1 fragment of mouse antibody gene heavy chain IgHM; sgRNA for deleting mouse antibody gene light chain Igκc; and sgRNA for deleting a fragment between IgLc2 and IgLc1 of mouse antibody gene light chain Igλ; preferably, wherein the sgRNA for deleting the CH1 fragment of mouse antibody gene heavy chain IgHM comprises a sgRNA as shown in SEQ ID NO: 1 and a sgRNA as shown in SEQ ID NO: 2; and/or, the sgRNA for deleting the mouse antibody gene light chain Igκc comprises a sgRNA as shown in SEQ ID NO: 3; and/or, the sgRNA for deleting the fragment between IgLc2 and IgLc1 of mouse antibody gene light chain Igλ comprises a sgRNA as shown in SEQ ID NO: 4 and a sgRNA as shown in SEQ ID NO: 5.

15. A sgRNA composition, which comprises: sgRNA for deleting a fragment between IgHM and IgHA-CH1 of mouse antibody gene heavy chain; sgRNA for deleting mouse antibody gene light chain Igκc; and sgRNA for deleting a fragment between IgLc2 and IgLc1 of mouse antibody gene light chain Igλ;
preferably, wherein the sgRNA for deleting the fragment between IgHM and IgHA-CH 1 of mouse antibody gene heavy chain comprises a sgRNA as shown in SEQ ID NO:1 and a sgRNA as shown in SEQ ID NO:6; and/or, the sgRNA for deleting the mouse antibody gene light chain Igκc comprises a sgRNA as shown in SEQ ID NO: 3; and/or, the sgRNA for deleting the fragment between IgLc2 and IgLc1 of mouse antibody gene light chain Igλ comprises a sgRNA as shown in SEQ ID NO:4 and a sgRNA as shown in SEQ ID NO:5.
